# FASCICULE DE BREVET EUROPEEN

(11) **EP 0 586 471 B1**
(45) Date de publication et mention de la délivrance du brevet: **28.12.1994**
(21) Numéro de dépôt: 92911071.6
(22) Date de dépôt: 15.05.1992
(51) Int. Cl.: C07D 209/44

(54) **DERIVES DE PERHYDROISOINDOLE ET LEUR PREPARATION**
PERHYDROISOINDOL DERIVATE UND IHRE HERSTELLUNG
DERIVATIVES OF PERHYDROISOINDOLE AND PREPARATION THEREOF

(30) Priorité: 17.05.1991 FR 9106036
(43) Date de publication de la demande: 16.03.1994
(73) Titulaire: RHONE-POULENC RORER S.A., 92160 Antony (FR)
(72) Inventeur: ACHARD, Daniel, F-94320 Thiais (FR); GRISONI, Serge, F-94600 Choisy-le-Roi (FR); HANESSIAN, Stephen, Beaconsfields, Quebec H9W 5H3 (CA); MOUTONNIER, Claude, F-92350 Le Plessis-Robinson (FR); PEYRONEL, Jean-François, F-91120 Palaiseau (FR); TABART, Michel, F-75013 Paris (FR); TRUCHON, Alain, F-69003 Lyon (FR)
(74) Mandataire: Savina, Jacques
(86) Numéro de dépôt international: FR9200430
(87) Numéro de publication internationale: WO9220654

(56) Documents cités:
- EP-A- 0 058 567
- EP-A- 0 058 567
- EP-A- 0 068 822
- EP-A- 0 068 822
- EP-A- 0 093 805
- EP-A- 0 093 805
- EP-A- 0 359 172
- EP-A- 0 359 172
- AU-B- 502 760
- AU-B- 502 760
- US-A- 4 042 707
- US-A- 4 042 707

## Description

La présente invention concerne de nouveaux dérivés du perhydroisoindole de formule générale : 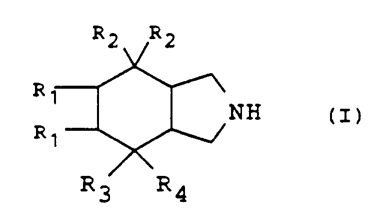
ainsi que leurs sels, qui sont des intermédiaires pour la préparation de perhydroisoindoles qui antagonisent les effets de la substance P et sont intéressants dans les domaines thérapeutiques où cette substance est connue pour intervenir.

Dans le brevet américain 4 O42 707 avaient été décrits des produits dérivés de l'isoindole de formule générale: 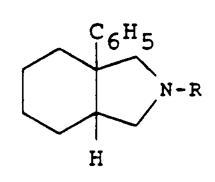
ayant une activité opiacée.

Ces produits n'ont pas d'activité vis à vis de la substance P et ne peuvent pas non plus être utilisés comme intermédiaires pour conduire à de tels produits.

Jusqu'à présent, malgré les recherches mises en oeuvre et malgré l'intérêt suscité [M.R. Hanley, TINS, (5) 139 (1982)], il n'avait pratiquement pas été découvert de produit agissant spécifiquement sur la substance P et ayant une structure non peptidique, c'est pourquoi les dérivés de l'isoindole de formule générale (I) présentent un intérêt considérable.

Dans la formule générale (I) :
- les radicaux R₁ sont identiques et représentent des atomes d'hydrogène ou forment ensemble une liaison,
- les symboles R₂ sont identiques et représentent des radicaux phényle éventuellement substitués par un atome d'halogène ou par un radical méthyle en position 2 ou 3,
- le symbole R₃ représente un atome d'halogène ou un radical hydroxy et
- le symbole R₄ représente un atome d'hydrogène ou simultanément à R₃ représente un atome d'halogène.

Lorsque R₂ porte un substituant halogène, ou lorsque R₃ est un atome d'halogène ,ce dernier peut être choisi parmi le chlore ou le fluor.

Par ailleurs, les produits de formule générale (I) présentent différentes formes stéréoisomères, il est entendu que les dérivés de l'isoindole de forme (3aR,7aR) à l'état pur, ou sous forme de mélange des formes cis (3aRS,7aRS), entrent dans le cadre de la présente invention. Lorsque les radicaux R₃ et R₄ sont différents, il est également entendu que le substituant R₃ peut être en position axiale ou équatoriale et de ce fait que les dérivés R et S ainsi que leurs mélanges entrent aussi dans le cadre de la présente invention.

Selon l'invention le dérivé de l'isoindole de formule générale (I) pour lequel R₃ représente un atome d'halogène et R₄ représente un atome d'hydrogène ou d'halogène, peut être obtenu par halogénation du dérivé de l'isoindole de formule générale : 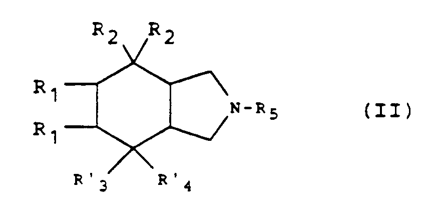
pour lequel R₁ et R₂ sont définis comme précédemment, R'₃ est un radical hydroxy, R'₄ est un atome d'hydrogène si l'on veut obtenir un dérivé monohalogéné, ou R'₃ et R'₄ forment ensemble un radical oxo si l'on veut obtenir un dérivé dihalogéné, puis élimination du radical protecteur R₅.

Le radical protecteur R₅ peut être tout groupement protecteur d'amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloacétyle, trifluoracétyle, vinyloxycarbonyle phénoxycarbonyle, chloro-1 éthoxycarbonyle ou chlorocarbonyle.

Lorsque l'on veut obtenir un produit pour lequel R₃ représente un atome de fluor, la réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme l'hexafluoropropyl diéthylamine (brevet japonnais 2 039 546) ou la N-(chloro-2 trifluoro-1,1,2 éthyl) diéthylamine, comme le tétrafluorure de sélenium (J. Am. Chem. Soc., 96,925 (1974) ou comme le tétrafluorophénylphosphorane (Tet. Let., 907 (1973), en opèrant dans un solvant organise tel qu'un solvant chloré (dichlorométhane, dichloréthane par exemple) à une température comprise entre -30 et +30°C. Il est entendu que la mise en oeuvre de l'alcool de configuration (S) conduit au dérivé fluoré de configuration (R) et que la mise en oeuvre de l'alcool de configuration (R) conduit au dérivé fluoré de configuration (S). Il est également possible d'opérer à partir d'un mélange des alcools de configuration (R) et (S) et d'effectuer la séparation au niveau du dérivé de formule générale (I) ainsi obtenu.

Lorsque l'on veut obtenir le dérivé difluoré de formule générale (I), la réaction s'effectue à partir de l'isoindolone de formule générale (II) (R'₃ et R'₄ forment ensemble un radical oxo), en opérant dans les conditions définies ci-dessus, à une température comprise entre 30°C et la température de reflux du mélange réactionnel.

Lorsque l' on veut obtenir un produit pour lequel R₃ représente un atome de chlore, le dérivé chloré de configuration (R) peut être obtenu par traitement de l'alcool (S) par le pentachlorure de phosphore dans les conditions définies par R.J. Cremlyn et coll., J. Chem. Soc., 3794 (1954) ; le dérivé chloré de configuration (S) peut être obtenu par traitement de l'alcool (S) par le chlorure de thionyle dans les conditions citées par R.J. Cremlyn dans la référence mentionnée ci-dessus.

Lorsque l'on veut obtenir le dérivé dichloré, on opère à partir de la perhydroisoindole de formule générale (II), par traitement par le pentachlorure de phosphore dans les conditions citées ci-dessus.

L'élimination subséquente du radical protecteur R₅ s'effectue selon les méthodes habituelles. Notamment, on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

Selon l'invention, le dérivé de l'isoindole de formule générale (I) pour lequel R₃ est un atome d'halogène et R₄ est un atome d'hydrogène, peut également être obtenu par halogénation d'un dérivé de perhydroisoindole de formule générale : 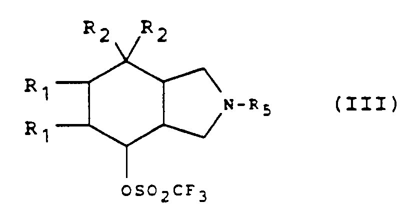
dans laquelle R₁, R₂ et R₅ sont définis comme précédemment, puis élimination du radical protecteur R₅.

L'halogénation s'effectue par un halogénure d'ammonium quaternaire tel que par exemple fluorure de tétrabutylammmonium ou par un halogénure alcalin tel que le fluorure de potassium ou le fluorure de césium par exemple, en milieu anhydre, dans un solvant organise tel qu'un éther (tétrahydrofuranne, dioxanne par exemple) un solvant chloré (dichlorométhane par exemple) ou dans un mélange de solvants, à une température comprise entre -30 et 50°C.

Il est entendu que le dérivé sulfonylé de formule générale (III) de configuration (S) conduit à un dérivé halogéné de configuration (R) et que le dérivé sulfonylé de configuration (R) conduit à un dérivé halogéné de configuration (S).

L'élimination du radical R₅ s'effectue comme décrit précédemment.

Le dérivé sulfonylé de formule générale (III) peut être obtenu par traitement du dérivé de perhydroisoindole de formule générale (II) pour lequel R'₃ est un radical hydroxy et R'₄ est un atome d'hydrogène, par un dérivé réactif de l'acide trifluorométhane sulfonique.

La réaction s'effectue généralement par action de l'anhydride trifluorométhane sulfonique en présence de pyridine, dans un solvant chloré (dichlorométhane par exemple), à une température comprise entre -30 et 20°C.

Selon l'invention, le dérivé de perhydroisoindole de formule générale (I) pour lequel R₃ est un radical hydroxy et R₄ est un atome d'hydrogène, peut être obtenu par réduction du dérivé de la perhydroisoindolone de formule générale : 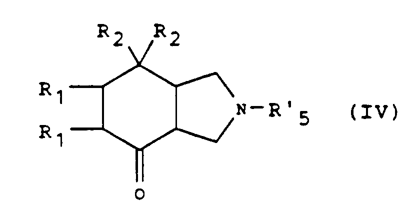
dans laquelle R₁ et R₂ sont définis comme précédemment et R'₅ est défini comme R₅ ou représente un atome d'hydrogène, suivie de la séparation des isomères axial et équatorial et/ou suivie de l'élimination du radical protecteur lorsque R'₅ est autre que l'atome d'hydrogène.

La réduction s'effectue avantageusement au moyen d'un borohydrure alcalin (borohydrure de sodium, tri-s.butylborohydrure de lithium) dans un solvant tel qu'un alcool (méthanol, éthanol par exemple) ou un éther (tétrahydrofuranne) en milieu basique ou par un aluminohydrure (hydrure d' aluminium et de lithium par exemple), à une température comprise entre -20 et 50°C.

L'élimination du radical R'₅ s'effectue selon les méthodes connues qui n'affectent pas le reste de la molécule.

Selon l'invention, le dérivé hydroxylé de perhydroisoindole de formule générale (I) dans laquelle R₃ est un radical hydroxy et R₄ est un atome d'hydrogène peut être aussi obtenu par libération du radical protecteur R₅ du dérivé de perhydroisoindole correspondant de formule générale (II) dans laquelle R'₃ et R'₄ sont définis comme ci-dessus.

L'élimination s'effectue selon les méthodes connues qui n'affectent pas le reste de la molécule, notamment selon les méthodes citées précédemment.

Le dérivé de perhydroisoindole de formule générale (II), ou le dérivé de perhydroisoindole de formule générale (IV) pour lequel R'₅ est défini comme R₅ peut être préparé par protection de l' amino du dérivé correspondant de formule générale : 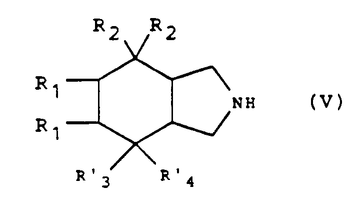
dans laquelle R₁, R₂, R'₃ et R'₄ sont définis comme pour la formule générale (II).

La protection s'effectue selon les méthodes habituelles. Notamment selon les références citées précédemment.

Le dérivé de l'isoindole de formule générale (IV) pour lequel R'₅ est un atome d'hydrogène, ou (V) pour lequel R'₃ et R'₄ forment ensemble un radical oxo, peut être obtenu à partir du dérivé correspondant de formule générale : 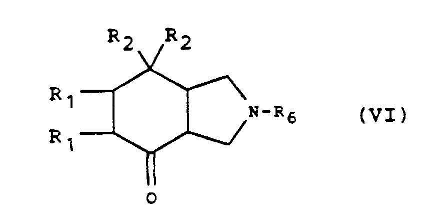
dans laquelle R₁ et R₂ sont définis comme précédemment et R₆ représente un radical allyle ou un radical de structure - CRₐR_{b}R_{c} dans laquelle Rₐ et R_{b} sont des atomes d' hydrogène ou des radicaux phényle éventuellement substitués (par un atome d'halogène, un radical alcoyle, alcoyloxy ou nitro), et R_{c} est défini comme Rₐ et R_{b} ou représente un radical alcoyle ou alcoyloxyalcoyle, l'un au moins de Rₐ, R_{b} et R_{c} étant un radical phényle substitué ou non et les radicaux alcoyle contenant 1 à 4 atomes de carbone en chaîne droite ou ramifiée, par élimination du radical R₆, par toute méthode connue qui n'affecte pas le reste de la molécule.

Notamment, lorsque R₁ est un atome d'hydrogène, et lorsque R₆ est autre qu'un radical allyle, le groupement R₆ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque R₆ est un radical benzhydryle ou trityle, l'élimination peut être effectuée par traitement en milieu acide, en opérant à une température comprise entre O°C et la température de reflux du mélange réactionnel, dans un alcool, dans un éther, dans l'eau ou directement dans l'acide acétique, l'acide formique ou l'acide trifluoroacétique.

Le groupement R₆ peut être également éliminé en faisant agir le chloroformiate de vinyle, le chloroformiate de chloro-1 éthyle ou le chloroformiate de phényle, en passant intermédiairement par un produit de formule générale : 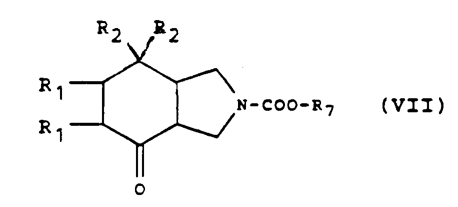
dans laquelle R₁ et R₂ sont définis comme précédemment, et R₇ est un radical vinyle, chloro-1 éthyle ou phényle, puis par élimination du radical -COOR₇ Par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en opérant à une température comprise entre 20°C et la température de reflux du mélange réactionnel.

L'élimination du radical -COOR₇ est effectuée par traitement en milieu acide par exemple par l' acide trifluoroacétique, formique, méthanesulfonique, p.toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux -COOR₇ citées précédemment, le dérivé de l'isoindolone de formule générale (IV) ou (V) attendu est obtenu à l'état de sel de l'acide employé qui peut être mis en oeuvre directement dans l'étape ultérieure.

Le dérivé de l'isoindolone de formule générale (VI) peut être obtenu par réaction de cycloaddition, par action d'un dérivé silylé de formule générale : 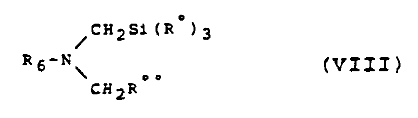
dans laquelle R₆ est défini comme précédemment, (R°)₃ représente des radicaux alcoyle Ou des radicaux alcoyle et phényle et R°° représente un radical alcoyloxy, cyano ou phénylthio, sur le dérivé de la cyclohexènone de formule générale : 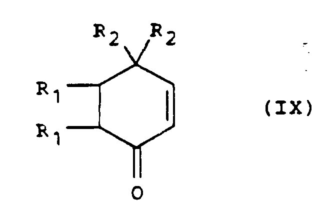
dans laquelle R₁ et R₂ sut définis corne précédemment.

On opère en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre O°C et la température de reflux du mélange réactionnel.

Le dérivé silylé de formule générale (VIII) peut être obtenu selon les méthodes décrites par :
- Y. Terao et coll., Chem. Pharm. Bull., 33, 2762 (1985);
- A. Hosomi et coll., Chem. Lett., 1117 (1984)
- A. Padwa et coll., Chem. Ber., 119, 813 (1986) ou
- Tetrahedron, 41, 3529 (1985).

Il est entendu que les dérivés de perhydroisoindole de formule générale (I), (II), (III), (IV), (V), (VI), et (VII) présentent plusieurs formes stéréoisomères. Lorsque l'on veut obtenir un produit de formule générale (I) de forme (3aR,7aR), la séparation des formes isomères peut être mise en oeuvre au niveau du dérivé de formule générale (V) pour lequel R'₃ et R'₄ forment ensemble un radical oxo. Elle peut aussi être mise en oeuvre au niveau du dérivé de formule générale (I). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l' acide dibenzoyltartrique, puis séparation des isomères par cristallisation. L'isomère recherché est libéré de son sel en milieu basique.

La séparation des isomères axial et équatorial des dérivés hydroxylés ou des dérivés halogénés s'effectue avantageusement au niveau des produits de formule générale (II) ou (V), en opérant par cristallisation et/ou chromatographie. Il est également possible d'opérer au niveau des produits de formule générale (I).

Selon l'invention, les dérivés de l'isoindole de formule générale (I) peuvent être utilisés pour la préparation de dérivés de formule générale : 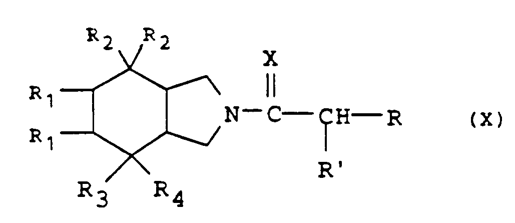
dans laquelle
- le symbole X représente un atome d'oxygène, ou un radical NH,
- le symbole R représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole R' représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino, et
   les symboles R₁, R₂, R₃ et R₄ sont définis comme pour la formule générale (I);
   les radicaux alcoyle ou acyle cités ci-dessus contennant 1 à 4 atomes de carbone en chaîne droite ou ramifiée;
   lorsque R contient un atome d'halogène, ce dernier étant choisi parmi le chlore, le brome, le fluor ou l'iode;
   lorsque R représente un radical hétérocyclyle mono ou polycyclique, saturé ou insaturé, celui-ci pouvant être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle;
   lorsque R représente phényle substitué par une chaîne portant un hétérocycle, ce dernier pouvant être choisi parmi pyrrolidinyle, morpholino, pipéridinyle, tétrahydropyridinyle pipérazinyle, ou thiomorpholino.

De plus, lorsque le symbole R' est autre que l'atome d'hydrogène la chaîne substituée sur l'isoindole présente un centre chiral, il est entendu que les formes stéréoisomères et leurs mélanges entrent aussi dans la formule générale (X).

Selon l'invention les dérivés du perhydroisoindole de formule générale (X) peuvent être obtenus par action de l'acide de formule générale : 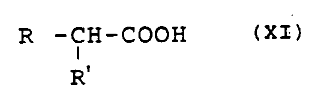
ou d'un dérivé réactif de cet acide, dans lequel R et R' sont définis comme précédemment, sur un dérivé de l'isoindole de formule générale (I) dans laquelle les symboles R₁, R₂, R₃ et R₄ sont définis comme précédemment suivie le cas échéant de la transformation de l'amide obtenu en une amidine .

Il est entendu que, les radicaux amino, alcoylamino ou carboxy contenus dans R et/ou R' sont de préférence préalablement protégés. La protection s'effectue par tout groupement compatible, dont la mise en place et l'élimination n'affectent pas le reste de la molécule. Notamment selon les méthodes décrites par T.W. Greene, par A. Wiley ou par Mc Omie dans les références citées précédemment.

A titre d'exemple,
- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque R' représente un radical hydroxy, il est préférable de protéger préalablement ce radical. La protection s'effectue par exemple par un radical acétoxy, trialcoylsilyle, benzyle, ou sous forme d'un carbonate par un radical -COORa dans lequel Ra est un radical alcoyle ou benzyle.

Lorsque l'on effectue la condensation d'un dérivé réactif de l'acide de formule générale (XI), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, di-nitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl) -1 éthyl-3 carbodiimide], le NN'-carbonyldiimidazole ou l'éthoxy-2 éthoxycarbonyl-1 dihydro-1,2 quinoléine ou bien en milieu hydroorganique, en présence d'un agent alcalin de condensation comme le bicarbonate de sodium, puis on transforme le cas échéant l'amide obtenu en une amidine telle que définie précédemment.

La transformation de l'amide de formule générale (X) en une amidine pour laquelle X est un radical NH s'effectue en préparant le dérivé de l'isoindolium de formule générale : 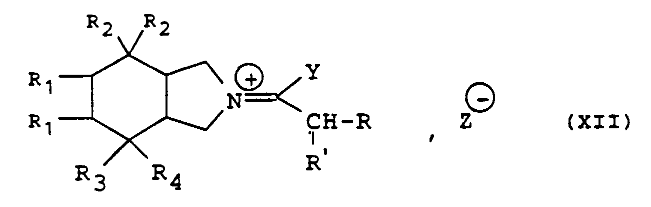
dans laquelle R, R', R₁, R₂, R₃ et R₄ sont définis comme précédemment, Y représente un atome de chlore, un radical méthoxy ou éthoxy et Z⁻ représente un ion chlorure, tétrafluoroborate, fluorosulfonate, trifluorométhylsulfonate, méthylsulfate, ou éthylsulfate, puis par action de l'ammoniac sur le dérivé de l'isoindolium.

Il est entendu que, lorsque R₃ est hydroxy, Y est autre qu'un atome de chlore.

La préparation du dérivé de l'isoindolium de formule générale (XII) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformiate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La réaction s'effectue dans un solvant chloré (dichlorométhane, dichloréthane par exemple) ou dans un hydrocarbure aromatique (toluène par exemple), à une température comprise entre O°C et la température de reflux du mélange réactionnel. L'action de l'ammoniac sur le dérivé de formule générale (XII) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -20°C et la température de reflux du mélange réactionnel.

Il n'est pas indispensable d'avoir isolé le dérivé de l'isoindolium de formule générale (XII) pour le mettre en oeuvre dans cette réaction.

Les dérivés de l' isoindole de formule générale (X) pour lesquels X est un radical imino peuvent également être obtenus à partir du dérivé de l'isoindole selon l'invention, par action d'un produit de formule générale : 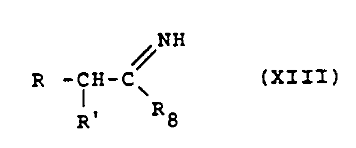
éventuellement à l'état de sel, dans laquelle R et R' sont définis comme précédemment et R₈ représente un radical alcoyloxy contenant 1 à 4 atomes de carbone en chaîne droite ou ramifiée ou un radical méthylthio, éthylthio, benzylthio ou alcoyloxycarbonylméthylthio.

La réaction est mise en oeuvre au moyen du dérivé de formule générale (XIII) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l' acétonitrile à une température comprise entre O°C et la température de reflux du mélange réactionnel.

Il est entendu que, dans l'éventualité où les radicaux R et/ou R' du produit de formule générale (XIII) portent des substituants pouvant interférer avec la réaction, ces derniers doivent être préalablement protégés.

Les acides de formule générale (XI) sont préparés selon les méthodes connues ou décrites ci-après dans les exemples, ou par analogie avec ces méthodes.

Les nouveaux dérivés de l'isoindole de formule générale (I) et les produits de formule générale (X) auquels ils conduisent peuvent être purifiés le cas échéant par des méthodes physiques telles que la cristallisation ou la chromatographie.

Le cas échéant, les nouveaux dérivés de formule générale (I), ainsi que les produits de formule générale (X) auquels ils conduisent pour lesquels les symboles R et/ou R' contiennent des substituants amino ou alcoylamino et/ou X représente un radical NH, peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindole de formule générale (X) antagonisent les effets de la substance P et peuvent ainsi trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 5 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S, Rosell et coll., Substance P, Ed. by US Von Euler and B. Pernow, Raven Press, New-York (1977), pages 83 à 88, les produits étudiés se sont montrés actifs à des doses comprises entre 20 et 1000 nM.

La substance P est connue pour être impliquée dans un certain noire de domaines pathologiques :
- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiovascular Pharmacology, 10 (suppl. 12), 5172 (1987).

Par ailleurs, les dérivés de l'isoindole de formule générale (X) ne présentent Pas de toxicité, ils se sont montrés atoxiques chez la souris par voie sous cutanée à la dose de de 40 mg/kg ou par voie orale à la dose de 100 mg/kg.

D'un intérêt particulier sont les dérivés de perhydroisoindole de formule générale (I) pour lesquels :
- les radicaux R₁ sont des atomes d'hydrogène,
- les symboles R₂ sont identiques et représentent des radicaux phényle,
- le symbole R₃ représente un atome de fluor ou de chlore ou un radical hydroxy et
- le symbole R₄ représente un atome d'hydrogène ou simultanément à R₃ représente un atome de fluor.

Et parmi ces produits plus spécialement intéressants sont les produits suivants :
- diphényl-7,7 perhydroisoindolol-4,
- diphényl-4,4 fluoro-7 perhydroisoindole,
- diphényl-4,4 difluoro-7,7 perhydroisoindole,
- chloro-7 diphényl-4,4 perhydroisoindole,
sous leurs formes stéréoisomères ainsi que leurs mélanges et leurs sels.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

### Exemple 1

A une solution de 100 g de chlorhydrate de diphényl-7,7 perhydroisoindolone-4-(3aR,7aR) dans 1000 cm³ de méthanol absolu refroidie à 5°C on ajoute en 90 minutes une solution de 7,18 g de borohydrure de sodium dans 500 cm³ de méthanol additionnée de 20 gouttes de solution concentrée de soude. Après agitation pendant 2,5 heures entre 5 et 10°C, les cristaux formés sont essorés et repris par 900 cm³ d'eau et 1000 cm³ d'éther éthylique. La solution est filtrée et alcalinisée par 15 cm³ de solution de soude 4N puis agitée 2 heures à 5°C. Les cristaux formés sont essorés, lavés à l'éther éthylique et séchés pour donner 28,8 g de diphényl-7,7 perhydroisoindolol-4-(3aR,7S,7aR) sous la forme de cristaux blancs fondant à 205°C, [α]_{D}²⁰= - 230° (c=1, CHCl₃).

Le chlorhydrate de diphényl-7,7 perhydroisoindolone-4-(3aR,7aR) peut être préparé de la manière suivante :
A une suspension de 200 g de chlorhydrate de diphényl-7,7 perhydroisoindolone-4 (3aRS,7aRS) dans 2000 cm³ d'acétate d'éthyle, on ajoute lentement, sous agitation, 500 cm³ de soude aqueuse 4N ; l'agitation est poursuivie jusqu'à dissolution du produit de départ. La solution organique est lavée par 250 cm³ d'eau distillée, par 250 cm³ d'une solution aqueuse saturée en chlorure de sodium, séchée sur sulfate de magnésium et filtrée. A la solution ainsi obtenue, on ajoute, sous agitation, une solution de 92,8 a d'acide L-(+) mandélique dans 1000 cm³ d'acétate d'éthyle : après 4 heures d'agitation, les cristaux obtenus sont essorés, lavé par 250 cm³ d'acétate d'éthyle (2 fois) et séchés. Les cristaux sont repris pas 2000 cm³ d'eau distillée ; le mélange est chauffé au reflux, sous agitation, pendant 15 minutes ; les cristaux insolubles sont essorés, lavés par 100 cm³ d eau distillée (2 fois ) et séchés. Ils sont recristallisés dans un mélange de 1100 cm³ d'acétonitrile et de 500 cm³ d'eau distillée; les cristaux obtenus sut essorés, lavés par 40 cm³ d'acétonitrile (3 fois) et séchés. On obtient 80 g de (L)-mandélate de diphényl-7,7 perhydroisoindolone-4 (3aR,7aR); [α]_{D}²⁰ = -164° (c=1, méthanol).

A 80 g de (L)-mandélate de diphényl-7,7 perhydroisoindolone-4 (3aR,7aR), on ajoute 400 cm³ de soude aqueuse 1N et 600 cm³ d'acétate d'éthyle ; le mélange est agité à température ambiante jusqu'à dissolution du produit de départ ; la solution organique est lavée par 250 cm³ d'eau distillée, par 250 cm³ d'une solution aqueuse saturée en chlorure de sodium, séchée sur sulfate de magnésium et filtrée ; elle est acidifiée, sous agitation, par addition de 30 cm³ d'acide chlorhydrique 9N : les cristaux obtenus sont essorés, lavés par 50 cm³d'acétate d'éthyle (2 fois), par 50 cm³ d'oxyde d'isopropyle et séchés. On obtient 52,3 g de chlorhydrate de diphényl-7,7 perhydroisoindolone-4 (3aR,7aR), fondant à 270°C avec décomposition; [α]_{D}²⁰₌ -282° (c=0,5, méthanol).

Le chlorhydrate de diphényl-7,7 perhydroisoindolone-4-(3aRS,7aRS) peut être préparé selon la méthode suivante :
A 15 g de palladium sur charbon à 10%, on ajoute 150 g de benzyl-2 diphényl-7,7 perhydroisoindolone-4 (3aRS, 7aRS), 1500 cm³ de méthanol et 450 cm³ d'acide chlorhydrique 1N ; le mélange réactionnel est hydrogéné, sous agitation, à température ambiante et sous pression atmosphérique. Après 5 heures de réaction le volume théorique d'hydrogène a été absorbé ; le mélange réactionnel est filtré, puis concentré à sec sous pression réduite (2,7 kPa) ; le résidu est cristallisé dans 200 cm³ d'éthanol ; les cristaux obtenus sont essorés, lavés par 50 cm³ d'éthanol et séchés. On obtient 110 g de chlorhydrate de diphényl-7,7 perhydro-isoindolone-4-(3aRS,7aRS) fondant à 270°C avec décomposition.
Spectre de RMN du proton :
2,O3 (Mt, 1H, 1H du H en 5 ou 6) ; 2,3 (Mt, 1H, 1H du - H en 5 ou 6) ; 2,48 (DD, en partie masqué, 1H du -CH₂- en 1) ; 2,69 (DD, 1H, 1H du -CH₂- en 1); 2,8 (Mt, 2H, -CH₂- en 6 ou 5) ; 3,34 (DD, en partie masqué, 1H du -CH₂- en 3) ; 3,5 (Mt, 1H, -CH- en 3a) : 3,82 (DD, 1H, 1H du -CH₂- en 3) : 3,95 (Mt, 1H, -CH- en 7a) : 7,15 à 7,65 (Mt, 10H, aromatiques) : 9,43 (Mf, 2H, -NH₂⁺).
Spectre infra-rouge (KBr) bandes caractéristiques en cm⁻¹:
3600-3300, 3100-3000, 3000-2850, 3100-2400, 1715, 1595, 1580, 1495, 1470, 1445, 775, 750, 705.

La benzyl-2 diphényl-7,7 perhydroisoindolone-4-(3aRS,7aRS) peut être préparée de la manière suivante :
A une solution de 155 g de diphényl-4,4 cyclohexène-2 one-1 et de 202 cm³ de N-butoxyméthyl N-triméthylsilylméthyl benzylamine dans 1000 cm³ de dichlorométhane sec, on ajoute 5 gouttes d'acide trifluoroacétique et chauffe le mélange réactionnel au reflux pendant 45 minutes. On ajoute 50 cm³ de N-butoxyméthyl N-triméthylsilylméthyl benzylamine et 3 gouttes d'acide trifluoroacétique et agite encore 45 minutes au reflux avant d'ajouter de nouveau 25 cm³ de N-butoxyméthyl N-triméthylsilylméthyl benzylamine et 3 gouttes d'acide trifluoroacétique. Le mélange réactionnel est agité au reflux 45 minutes puis traité par 50 g de carbonate de potassium, filtré et concentré à sec sous pression réduite (2,7 kPa). Le résidu est dissous dans 200 cm³ d'oxyde d'isopropyle et la solution refroidie à 0°C pendant 1 heure. Les cristaux sont essorés, lavés 2 fois par 15 cm³ d'oxyde d'isopropyle et séchés pour donner 193 g de benzyl-2 diphényl-7,7 perhydroisoindolone-4-(3aRS,7aRS) sous la forme de cristaux blancs fondant à 132°C.

La N-butoxyméthyl N-triméthylsilylméthyl benzylamine peut être préparée selon la méthode de Y. Terao et coll., Chem. Pharm. Bull., 33, 2762 (1985).

### Exemple 2

A une solution refroidie à +4°C de 17,8 g de diphényl-7,7 tert-butyloxycarbonyl-2 perhydroisoindolone-4-(3aR,7aR) dans un litre de méthanol on ajoute goutte à goutte pendant 40 minutes une solution de 1 g de borohydrure de sodium dans 200 cm³ de méthanol puis 10 gouttes de lessive de soude. Le mélange réactionnel est agité 3 heures à +4°C, puis on ajoute 2 cm³ d'une solution aqueuse d'acide chlorhydrique 0,1 N et on concentre à sec sous pression réduite (2,7 kPa). Le résidu est dissous dans 350 cm³ de dichlorométhane, lavé par 100 cm³ d'eau, puis par 50 cm³ de solution saturée de chlorure de sodium, séché sur sulfate de magnésium, concentré à sec sous pression réduite (2,7 kPa). Le résidu est cristallisé dans 40 cm³ d'éther éthylique. Les cristaux obtenus sont essorés et séchés. On obtient 8,4 g de diphényl-7,7 tert-butyloxycarbonyl-2 perhydroisoindolol-4-(3aR,4S,7aR) sous la forme de cristaux blancs fondant à 190°C. Les eaux mères de cristallisation sont concentrées à sec sous pression réduite (2,7 kPa). Le résidu est chromatographié sur colonne de gel de silice (granulométrie 0,04-0,06 mm, diamètre 4 cm, hauteur 33 cm), en éluant sous une pression de 0,4 bar d' azote par un mélange de dichlorométhane et de méthanol (96/4 en volumes) et en recueillant des fractions de 20 cm³. Les fractions 18 à 21 sont réunies et concentrées à sec sous pression réduite (2,7 kpa). On obtient 1,88 g de diphényl-7,7 tert-butyloxycarbonyl-2 perhydroisoindolol-4-(3aR,4R,7aR) sous forme de meringue blanche. Les fractions 26 à 31 sont réunies et concentrées à sec sous pression réduite (2,7 kPa). Le résidu est cristallisé dans 5 cm³ d éther éthylique. On obtient 2,88 g supplémentaires de diphényl-7,7 tert-butyloxycarbonyl-2 perhydroisoindolol-4-(3aR,4S,7aR) sous forme de cristaux blancs fondant à 190°C.

Le diphényl-7,7 tert-butyloxycarbonyl-2 perhydroisoindolone-4-(3aR,7aR) put être obtenu de la manière suivante :

A une solution de 20 g de chlorhydrate de diphényl-7,7 perhydroisoindolone-4-(3aR,7aR) dans 100 cm³ de dichlorométhane sec et 6,17 cm³ de triéthylamine on ajoute successivement 0,74 g de diméthylamino-4 pyridine, puis 14,7 g de di-tert-butyl dicarbonate. Le mélange réactionnel est agité pendant 24 heures à température ambiante, puis lavé par une solution aqueuse d'acide citrique, puis par une solution aqueuse d'hydrogénocarbonate de sodium, séché sur sulfate de magnésium, filtré et concentré à sec sous pression réduite (2,7 kPa). Le résidu est cristallisé dans 90 cm³ d'éther éthylique. Les cristaux sont essorés, lavés par 10 cm³ d'éther éthylique puis séchés. On obtient 14,1 g de diphényl-7,7 tert-butyloxycarbonyl-2 perhydroisoindolone-4-(3aR,7aR), sous la forme de cristaux blancs fondant à 119°C.

A une solution de 2 g de diphényl-7,7 tert-butyloxycarbonyl-2 perhydroisoindolol-4-(3aR,4S,7aR) dans 20 cm³ de dioxanne on ajoute une solution de 40 cm³ de dioxanne chlorhydrique 6,3 N et on agite à température ambiante pendant 5 heures. Le mélange réactionnel est concentré à sec sous pression réduite (2,7 kPa), trituré dans l'acétonitrile, filtré et séché. On obtient 1,57 g de chlorhydrate de diphényl-7,7 perhydroisoindolol-4-(3aR,4S,7aR) sous forme de cristaux blancs fondant à 266°C.

### Exemple 3

Le chlorhydrate de diphényl-7,7 perhydroisoindolol-4-(3aR,4R,7aR) peut être préparé par hydrogénation d'une suspension de 0,70 g de benzyl-2 diphényl-7,7 perhydroisoindolol-4-(3aR,4R,7aR) dans 30 cm³ de méthanol et 2,0 cm³ d'acide chlorhydrique 1N à pression atmosphérique pendant 20 heures à 20°C en présence de 0,12 g d'hydroxyde de palladium à 20% sur noir de carbone. Le mélange réactionnel est filtré, concentré à sec sous pression réduite (2,7 kPa), l'huile obtenue est concrètée avec de l'éther éthylique. La suspension est filtrée, le solide essoré et séché sous pression réduite (2,7 kPa). On obtient 0,52 g de chlorhydrate de diphényl-7,7 perhydroisoindolol-4-(3aR,4R,7aR), sous la forme d'un solide blanc fondant à 220°C ( avec décomposition).
Spectre infra-rouge (bandes caractéristiques, cm⁻¹): 3400, 3090, 3050, 3025, 3000-2800, 1600, 1580, 1495, 1465, 985, 750, 700.
Spectre RMN du proton (DMSO-d₆, signaux principaux) = 1,06 (t large, J=14, 1H, H en 5); 1,66 (d large, J=14, 1H, H en 5); 2,17 (d large, J=14, 1H, CH₂ en 6); 3,8 (s large, 1H, H en 4); 5,3 (mf, 1H, OH); 7,05 à 7,45 (mt, 10H, aromatiques); 8,4 et 9,43 (mf, 2H, NH₂⁺).

Le benzyl-2 diphényl-7,7 perhydroisoindolol-4-(3aR,4R,7aR) peut être préparé de la façon suivante:
A une solution refroidie à 0°C de 1,3 g de benzyl-2 diphényl-7,7 perhydroisoindolone-4-(3aR,7aR) dans 6,0 cm³ de tétrahydrofuranne, on ajoute en 5 minutes 4,0 cm³ d'une solution 1M de tri-sec-butyl borohydrure de lithium dans le tetrahydrofuranne. Le mélange réactionnel après agitation 3 heures à 0°C est à nouveau additionné de 0,5 cm³ de la solution 1M de borohydrure. Après 1 heure à 0°C, puis ajout de 50 cm³ d'eau et 50 cm³ d'acétate d'éthyle, la phase organique est décantée, lavée par 20 cm³ d eau, séchée sur sulfate de magnésium et concentrée à sec sous pression réduite (2,7 kPa). L'huile obtenue est cristallisée dans 30 cm³ d'oxyde de diisopropyle, les cristaux sont essorés, séchés sous pression réduite (2,7 kPa). On obtient 0,70 g de benzyl-2 diphényl-7,7 perhydroisoindolol-4-(3aR,4R,7aR), sous la forme de cristaux blancs fondant à 154°C.

Le banzyl-2 diphényl-7,7 perhydroisoindolone-4-(3aR,7aR) peut être préparé de la façon suivante:
A une solution refroidie à 0°C de 21,7 g de chlorhydrate de diphényl-7,7 perhydroisoindolone-4-(3aR,7aR), dans 300 cm³ de di-chlorométhane, et 18,5 cm³ de triéthylamine, on ajoute 7,9 cm³ de bromure de benzyle. Le mélange réactionnel après agitation 1 heure à 0°C et 2 heures à 20°C est lavé par 50 cm³ d eau, séché sur sulfate de magnésium, concentré à sec sous pression réduite (2,7 kPa). Le résidu est chromatographié sur une colonne de gel de silice (0,04-0,06 mm,diamètre 5 cm, hauteur 40 cm) en éluant sous une pression de 0,6 bar d'azote, par un mélange d'acétate d'éthyle et de cyclohexane (75/25 en volumes) et en recueillant des fractions de 250 cm³. Les fractions 3 à 6 sont réunies et concentrées à sec sous pression réduite (2,7 kPa). On obtient 22,1 g de benzyl-2 diphényl-7,7 perhydroisoindolone-4-(3aR,7aR), sous la forme d'un solide blanc fondant à 124°C. [a]²⁰D = -279°.

### Exemple 4

A une solution refroidie à +5°C de 1,0 g de tertiobutyl-oxycarbonyl-2 diphényl-7,7 perhydroisoindolol-4- (3aR,4R,7aR) dans 20 cm³ de dichlorométhane sec, on ajoute une solution de 0,37 cm³ de trifluorothio-4 morpholine dans 10 cm³ de dichlorométhane sec. Après 2 heures d'agitation à +5°C, le mélange réactionnel est lavé par 20 cm³ de solution aqueuse de bicarbonate de sodium à 5% puis séché sur sulfate de magnésium et concentré à sec sous pression réduite (2,7 kPa). Le résidu est chromatographié sur une colonne de gel de silice (granulométrie 0,04 mm - 0,06 mm, diamètre 2,4 cm, hauteur 35 cm), en éluant sous une pression d'azote de 0,8 bar, par un mélange de cyclohexane et d'acétate d'éthyle (90/10 en volumes) et en recueillant des fractions de 25 cm³. Les fractions 25 à 34 sont réunies et concentrées à sec. On obtient 0,27 g de diphényl-4,4 fluoro-7 tertiobutyloxycarbonyl-2 perhydroisoindole-(3aR,7S,7aR) sous la forme d'une meringue blanche.
Spectre infra-rouge (KBr, bandes caractéristiques, cm⁻¹): 3090, 3060, 3030, 2975, 2930, 2875, 1695, 1595, 1580, 1495, 1450, 1405, 1365, 1175, 755, 730, 700.

En opérant comme à l'exemple 8 ci-après à partir de 0,5 g de diphényl-4,4 fluoro-7 tertiobutyloxycarbonyl-2 perhydroisoindole-(3aR,7S,7aR), on obtient 0,35 g de chlorhydrate de diphényl-4,4 fluoro-7 perhydroisoindole-(3aR,7S,7aR) sous la forme d'un solide gris.
Spectre infra-rouge (KBr, bandes caractéristiques, cm⁻¹ ): 3420, 3090, 3050, 3025, 2970, 2920, 2800-2250, 1590, 1580, 1495, 1460, 1445, 1060, 750, 730, 700.

### Exemple 5

A une solution refroidie à +5°C de 9,4 g de diphényl-7,7 tert-butyloxycarbonyl-2 perhydroisoindolol-4-(3aR,4S,7aR) dans 250 cm³ de dichlorométhane sec on ajoute une solution de 3,5 cm³ de trifluorure de morpholinosoufre dans 50 cm³ de dichlorométhane. Le mélange réactionnel est agité 4 heures à +5°C puis dilué avec 300 cm³ de dichlorométhane, lavé par 250 cm³ de solution aqueuse d'hydrogénocarbonate de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 42 cm), en éluant sous une pression de 0,5 bar d'azote par un mélange de cyclohexane et d'acétate d'éthyle (90/10 en volumes ) et en recueillant des fractions de 120 cm³ . Les fractions 13 à 17 sont réunies et concentrées à sec sous pression réduite (2,7 kPa). Le résidu est cristallisé dans du cyclohexane. On obtient 2,55 g de diphényl-4,4 fluoro-7 tert-butyloxycarbonyl-2 perhydroisoindole-(3aR,7A,7aR) sous forme de cristaux blancs fondant à 202°C.

A une solution de 3,7 g de diphényl-4,4 fluoro-7 tert-butyloxycarbonyl-2 perhydroisoindole-(3aR,7R,7aR) dans 40 cm³ de dioxanne on ajoute une solution de 40 cm³ de dioxanne chlorhydrique 6,3 N et on agite à température ambiante pendant 2 heures. Le mélange réactionnel est concentré à sec sous pression réduite (2,7 kPa), trituré dans de l'oxyde de diisopropyle, filtré et séché. On obtient 3,1 g de chlorhydrate de diphényl-4,4 fluoro-7 perhydroisoindole-(3aR,7R,7aR) sous forme de cristaux blancs fondant à 200°C avec décomposition .

### Exemple 6

A une solution refroidie à +4°C de 1 g de diphényl-7,7 tert-butyloxycarbonyl-2 perhydroisoindolol-4-(3aR,4S,7aR) dans 60 cm³ de chloroforme, on ajoute successivement 1,3 g de carbonate de calcium, puis 2 g de pentachlorure de phosphore et on agite à température ambiante pendant 20 heures. Le mélange réactionnel est ensuite filtré, dilué par 80 cm³ de chloroforme, lavé deux fois par 80 cm³ d'eau, séchée sur sulfate de magnésium et concentré à sec sous pression réduite (2,7 kPa). Le résidu est chromatographié sur colonne de gel de silice (granulométrie 0,04-0,06 mm, diamètre 2,5 cm, hauteur 34 cm), en éluant sous une pression de 0,4 bar d'azote par un mélange de cyclohexane et d'acétate d'éthyle (30/70 en volumes) et en recueillant des fractions de 20 cm^{3.} Les fractions 7 à 10 sont réunies et concentrées à sec sous pression réduite (2,7 kPa). On obtient 0,44 g de chloro-7 chlorocarbonyl-2 diphényl-4,4 perhydroisoindole-(3aR,7R,7aR) sous la forme d'un solide blanc.
Spectre infra-rouge (Solution CCl₄, bandes caractéristiques, cm⁻¹): 3090, 3065, 3035, 2930, 2855, 1745, 1600, 1585, 1495, 1450, 700.

Une solution de 0,4 g de chloro-7 chlorocarbonyl-2 diphényl-4,4 perhydroisoindole-(3aR,7R,7aR) dans 6 cm³ d'une solution aqueuse 1N d'acide chlorhydrique et 14 cm³ de tétrahydrofuranne est portée à 80°C sous agitation pendant 9 heures. Le mélange réactionnel est concentré à sec sous pression réduite (2,7 kPa). On obtient 0,35 g de chlorhydrate de chloro-7 diphényl-4,4 perhydroisoindole-(3aR,7R,7aR) sous forme de solide blanc.
Spectre infra-rouge (KBr, bandes caractéristiques, cm⁻¹): 3055, 3025, 3000, 2250, 1600, 1495, 1580, 1460, 1445, 1435, 760, 750, 735, 700.

### Exemple 7

Une solution de 1 g de tert-butyloxycarbonyl-2 diphényl-7,7 perhydroisoindolol-4-(3aR,4S,7aR) dans 10 cm³ de chlorure de thionyle est agitée 3 heures à 80°C. Le mélange réactionnel est ensuite concentré à sec sous pression réduite (2,7 kPa). On obtient 1,03 g de tert-butyloxycarbonyl-2 chloro-7 diphényl-4,4 perhydroisoindole(3aR,7S,7aR) sous la forme d'un solide, utilisé brut dans l'essai suivant.

A une solution de 1,03 g de tert-butyloxycarbonyl-2 chloro-7 diphényl-4,4 perhydroisoindole-(3aR,7S,7aR) dans 5 cm³ de dioxanne on ajoute 10 cm³ d une solution 6,3 N d'acide chlorhydrique dans le dioxanne. Le mélange réactionnel est agité à température ambiante pendant 2 heures, puis concentré à sec sous pression réduite (2,7 kPa). On obtient 0,84 g de chlorhydrate de chloro-7 diphényl-4,4 perhydroisoindole-(3aR,7S,7aR) sous forme d'un solide, utilisé brut dans l'essai suivant.

### Exemple 8

A une solution de 3,4 cm³ de trifluorure de diéthylamino soufre dans 20 cm³ de dichlorométhane sec, on ajoute une solution de 5,0 g de tertiobutyloxycarbonyl-2 diphényl-7,7 perhydroisoindolone-4-(3aRS,7aRS) dans 30 cm³ de dichlorométhane sec. Après 5 heures d'agitation au reflux et 20 heures à 20°C, le mélange réactionnel est lavé par 50 cm³ de solution aqueuse saturée de bicarbonate de sodium et par 50 cm³ d eau, puis séché sur sulfate de magnésium, et concentré à sec. Le résidu est chromatographié sur une colonne de gel de silice (granulométrie 0,04 - 0,06 mm, diamètre 2,8 cm, hauteur 35 cm), en éluant sous une pression d'azote de 0,8 bar par un mélange de cyclohexane et d'acétate d'éthyle (95/5 puis 90/10 en volumes) et en recueillant des fractions de 25 cm³ . Les fractions 24 à 52 sont réunies et concentrées à sec sous pression réduite (2,7 kPa). Le résidu est cristallisé dans de l'acétate d'éthyle et de l'oxyde de diisopropyle, les cristaux sont essorés puis séchés. On obtient 1,80 g de tertiobutyloxycarbonyl-2 diphényl-4,4 difluoro-7,7 perhydroisoindole-(3aRS,7aRS) sous la forme de cristaux blancs fondant à 162°C.

A 1,8 g de tertiobutyloxycarbonyl-2 diphényl-4,4 difluoro-7,7 perhydroisoindole-(3aRS,7aRS) on ajoute 20 cm³ de dioxanne et 20 cm³ d'acide chlorhydrique 6,3N. Après 20 heures d'agitation à température ambiante, la suspension blanche obtenue est concentrée à sec à 40°C sous pression réduite (2,7 kPa). Le résidu est lavé avec de l'oxyde de diisopropyle, le solide obtenu essoré, puis séché. On obtient 1,51 g de chlorhydrate de diphényl-4,4 difluoro-7,7 perhydroisoindole-(3aRS,7aRS) sous la forme d'un solide blanc.
Spectre infra-rouge (KBr, bandes caractéristiques, cm⁻¹): 3090, 3050, 3025, 2965, 2935, 2900, 2800-2250, 1595, 1580, 1495, 1465, 1445, 760, 730, 700.
Spectre RMN du proton (DMSO-d₆ + CF₃COOD): 1,2-1,55 et 2,12 (2mt, 2x1H, CH₂ en 6); 3-3,3 (mt, 1H, H en 7a); 3,58 (mt, 2H, CH₂ en 1); 3,76 (mt, 1H, H en 3a); 7,1 à 7,5 (mt, 10H, aromatiques).

### Exemple 9

Une solution de 4,87 g de t-butoxycarbonyl-2 diphényl-4,4 trifluorométhylsulfonyloxy-7 perhydroisoindole-(3aR,7S,7aR) dans 150 cm³ de dichlorométhane sec est traitée par 22,6 cm³ de solution 1M de fluorure de tétrabutylammonium dans le tétrahydrofuranne puis agitée 17 heures à 20°C et concentré à sec sous pression réduite (2,7 kPa). Le résidu est chromatographié sur une colonne de gel de silice (granulométrie 0,2-0,063 mm, diamètre 4,5 cm, hauteur 35 cm) en éluant sous une pression de 0,4 bar d'azote par un mélange de cyclohexane et d'acétate d'éthyle (75/25) et en recueillant des fractions de 20 cm³. Les fractions 28 à 38 sont réunies et concentrées à sec sous pression réduite (2,7 kPa) pour donner 1,44 g de t-butoxycarbonyl-2 diphényl-4,4 fluoro-7 perhydroisoindole-(3aR,7R,7aR) sous la forme de cristaux blancs fondant à 200°C, [α]_{D}²⁰= - 225° (c=1, CHCl₃).

Une solution de 2,25 g de t-butoxycarbonyl-2 diphényl-4,4 fluoro-7 perhydroisoindole-(3aR,7R,7aR) dans 25 cm³ de dioxanne est traitée par une solution 5,8 N d'acide chlorhydrique dans le dioxanne et agitée 2 heures à 20°C puis concentrée à sec sous pression réduite (2,7 kPa). Le résidu est concrèté par addition de 100 cm³ d'oxyde d'isopropyle, le solide est filtré et séché pour donner 1,8 g de chlorhydrate de diphényl-4,4 fluoro-7 perhydroisoindole-(3aR,7R,7aR) sous la forme d'une poudre crème.
Spectre RMN du proton (DMSO-d₆): 1,0-1,35 (mt, 1H du CH₂en 6) ; 4,9 (d large, J=50, 1H, CHF) ; 7,1 à 7,5 (mt, 14H, aromatiques) ; 9,05 et 9,9 (2 mf, 2x1H, NH₂⁺).

Le t-butoxycarbonyl-2 diphényl-4,4 trifluorométhylsulfonyloxy-7 perhydroisoindole-(3aR,7S,7aR) peut être obtenu de la manière suivante :
A une solution de 6,7 g de t-butoxycarbonyl-2 diphényl-7,7 perhydroisondolol-4-(3aR,4S,7aR) dans 100 cm³ de dichlorométhane sec refroidie à -30°C on ajoute 1,5 cm³ de pyridine puis, en 10 minutes, une solution de 3,2 g d'anhydride trifluorométhanesulfonique dans 25 cm³ de dichlorométhane sec. Le mélange réactionnel est agité 2 heures à -30°C puis dilué par 250 cm³ d eau et 100 cm³ de dichlorométhane. La phase organique est lavée par 200 cm³ de solution saturée de bicarbonate de sodium et par 200 cm³ de solution saturée de chlorure de sodium puis séchée et concentrée à sec sous pression réduite (2,7 kPa) pour donner 8,6 g de t-butoxycarbonyl-2 diphényl-4,4 trifluorométhylsulfonyloxy-7 perhydroisoindole-(3aR,7S,7aR) sous la forme d'une meringue jaune utilisée telle quelle dans la suite de la synthèse.

Le t-butoxycarbonyl-2 diphényl-7,7 perhydroisoindolol-4-(3aR,4S,7aR) peut être obtenu de la manière suivante :
A une solution de 13 g de diphényl-7,7 perhydroisoindolol-4--(3aR,4S,7aR) et 0,5 g de diméthylamino-4 pyridine dans 450 cm³ de dichlorométhane on ajoute 10,55 g de diterbutyldicarbonate. Après agitation durant 2 heures à 25°C, le mélange réactionnel est concentré à sec sous pression réduite (2,7 kPa) et le résidu est cristallisé dans 50 cm³ d'éther éthylique. On obtient 9 g de t-butoxycarbonyl-2 diphényl-7,7 perhydroisoindolol-4-(3aR,4S,7aR) sous la forme de cristaux blancs fondant à 190°C.

Les produits selon l'invention peuvent être mis en oeuvre pour la préparation des dérivés de l' isoindole de formule générale (X) comme dans les exemples d'utilisation ci-après.

### Exemple d'utilisation 1

A une solution de 0,72 g de chlorhydrate de diphényl-4,4 fluoro-7 perhydroisoindole-(3aR,7R,7aR), 0,5 g d'acide (diméthylamino-3 propoxy)-2 phénylacétique, 0,03 g d'hydroxy-1 benzotriazole dans 75 cm³ de dichlorométhane refroidie à + 4°C on ajoute en 10 minutes une solution de 0,5 g de (diméthylamino-3 propyl)-1 éthyl-3 carbodiimide dans 50 cm³ de dichlorométhane sec puis 0,37 cm³ de diisopropyléthylamine. Le mélange réactionnel est agité 3 heures à 0°C puis lavé 2 fois par 50 cm³ d'eau et 2 fois par 50 cm³ de solution saturée de chlorure de sodium. La phase organique est séchée sur sulfate de magnésium et concentrée à sec sous pression réduite (2,7 kPa). Le résidu est repris par 21 cm³ d'acide chlorhydrique 0,1 N, 50 cm³ d'éther diéthylique et 30 cm³ d'eau. La phase aqueuse est séparée et lyophilisée pour donner 0,85 g de chlorhydrate de [(diméthylamino-3 propoxy)-2 phényl]acétyl-2 diphényl-4,4 fluoro-7 perhydroisoindole-(3aR,7R,7aR), sous la forme d'un lyophilisat blanc.
Spectre infra-rouge (KBr, bandes caractéristiques, cm⁻¹): 3060, 3030, 2960, 2890, 2800, 2200, 1635, 1605, 1495, 1460, 1445, 1250, 755, 705.
Spectre RMN du proton (DMSO-d₆) (à température ambiante, on observe le mélange des deux rotamères):
0,95-1,35 et 1,8-2,1 (2mt, 2x1H, CH₂ en 6) ; 2,6-2,8 (mt, 6H, H(CH₃)₂) ; 3,9 et 4,05 (2mt, 2x1H, OCH₂) ; 4,8 et 4,85 (2d larges, J=50, 1H, CHF) ; 6,8 à 7,5 (mt, 14H, aromatiques).

### Exemple d'utilisation 2

A une solution refroidie à +5°C de 1 g de chlorhydrate de diphényl-4,4 fluoro-7 perhydroisoindole-(3aR,7R,7aR) et de 0,924 g d'acide {[(pyrrolidinyl-1)-3 propoxy-2]phényl}-2 acétique dans 40 cm³ de dichlorométhane sec, on ajoute 0,04 g d'hydrate d'hydroxybenzotriazole, puis 0,79 g chlorhydrate de (diméthylamino-3 propyl)-1 éthyl-3 carbodiimide et 0,51 cm³ de diisopropyléthylamine. Après 2,3 heures d'agitation à +5°C et 20 heures à 20°C, le mélange réactionnel est lavé 2 fois par 50 cm³ d'eau, séché sur sulfate de magnésium, puis concentré à sec à 40°C sous pression réduite (2,7 kPa). Le résidu est chromatographié sur colonne de gel de silice (granulométrie 0,04-0,06 mm, diamètre 2,4 cm, hauteur 31 cm ), en éluant sous une pression de 0,5 bar d'azote par un mélange d'acétate d'éthyle, d'acide acétique et d'eau (60/10/10 en volumes) et en recueillant des fractions de 25 cm³. Les fractions 11 à 31 sont réunies et concentrées à sec sous pression réduite (2,7 kPa). Le résidu est repris avec 20 cm³ de dichlorométhane, la solution est lavée par 20 cm³ de solution aqueuse d'hydroxyde de sodium 1N, puis est séchée sur sulfate de magnésium, et concentrée à sec. Ce lavage basique est effectué une nouvelle fois. On obtient 0,68 g de {{[(pyrrolidinyl-1)-3 propoxy-2]phényl}acétyl}-2 diphényl-4,4 fluoro-7 perhydro-isoindole-(3aR,7R,7aR) sous la forme d'un solide blanc.
Spectre Infra-rouge (KBr, bandes caractéristiques, cm⁻¹): 3085, 3055, 3035, 2950, 2875, 2785, 1640, 1600, 1495, 1455, 1440, 1245, 750, 700.
Spectre RMN du proton (DMSO-d₆ + CF₃COOD)= 1,1-1,45 (mt, 1H, 1H en 6); 1,9 (mt, 4H, 2CH₂ en 3 et 4 du pyrrolidino); 2,27 (mt, 1H, 1H en 5); 3,77 (d, J=10, 1H, H en 1); 4,03 (mt, 2H, OCH₂); 4,78 (d large, J=50, 1H, CHF); 7,1 à 7,5 (mt, 14H, aromatiques).

### Exemple d'utilisation 3

En opérant comme à l'exemple 9 ci-après, à partir de 0,16 g d'acide diméthylamino-2 phénylacétique et de 0,30 g de chlorhydrate de diphényl-4,4 fluoro-7 perhydroisoindole- (3aR,7S,7aR) on obtient 0,11 g de [(diméthylamino-2 phényl)acétyl]-2 diphényl-4,4 fluoro-7 perhydroisoindole- (3aR,7S,7aR) sous la forme d'une meringue blanche.
Spectre infra-rouge (KBr, bandes caractéristiques, cm⁻¹): 3090, 3060, 3030, 2940, 2875, 2825, 2770, 1645, 1595, 1580, 1495, 1450, 1420, 755, 730, 700.
Spectre RMN du proton (à température ambiante, on observe le mélange de deux rotamères) = 2,35 et 2,58 (2s, 6H, N(CH₃)₂), 4,2-4,6 (mt, 1H, CHF), 6,9-7,5 (mt, 14H, aromatiques).

### Exemple d'utilisation 4

A une solution de 0,57 g de bromhydrate d'acide (pyrrolidino-2) phénylacétique dans 20 cm³ de dichlorométhane sec, refroidie à 4°C, on ajoute 0,28 cm³ de triéthylamine et 0,32 g de carbonyldiimidazole. On agite une heure à + 4°C, puis on ajoute une solution de 0,67 g de chlorhydrate de diphényl-4,4 fluoro-7 perhydroisoindole-(3aR,7R,7aR) dans 20 cm³ de dichlorométhane sec et 0,28 cm³ de triéthylamine. Le mélange réactionnel est agité à température ambiante pendant 24 heures, puis dilué par 100 cm³ de dichlorométhane, lavé deux fois par 50 cm³ d'eau, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 38 cm), en éluant sous une pression de 0,5 bar d'azote par un mélange de cyclohexane et d'acétate d'éthyle (70/30 en volumes) et en recueillant des fractions de 20 cm³ . Les fractions 26 à 54 sont réunies et concentrées à sec sous pression réduite (2,7 kPa). Le résidu est cristallisé dans un mélange d'acétonitrile et d'oxyde de diisopropyle (25/75 en volumes). On obtient 0,16 g de diphényl-4,4 fluoro-7 [(pyrrolidino-2 phényl)-acétyl]-2 perhydroisoindole-(3aR,7R,7aR) sous forme de cristaux blancs fondant à 170°C.

### Exemple d'utilisation 5

A une solution de 0,19 g d'acide (diméthylamino-2 phényl)-acétique dans 15 cm³ de dichlorométhane sec, refroidie à + 4°C, on ajoute 0,17 g de carbonyldiimidazole. On agite une heure à + 4°C, puis on ajoute une solution de 0,35 g de chlorhydrate de chloro-7 diphényl-4,4 perhydroisoindole-(3aR,7R,7aR) dans 10 cm³ de di-chlorométhane sec, puis une solution de 0,15 cm³ de triéthylamine dans 10 cm³ de dichlorométhane sec. Le mélange réactionnel est agité à température ambiante pendant 20 heures, Dis dilué par 120 cm³ de dichlorométhane, lavé pas 80 cm³ d'eau, puis par une solution aqueuse saturée en chlorure de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 2 cm, hauteur 22 cm), en éluant sous une pression de 0,4 bar d'azote par un mélange d'acétate d'éthyle et de cyclohexane (75/25 en volumes) et en recueillant des fractions de 20 cm³ . Les fractions 6 à 9 sont réunies et concentrées à sec sous pression réduite (2,7 kPa). Le produit, obtenu sous forme de base, est converti en chlorhydrate par dissolution dans 25 cm³ d'éther éthylique, puis ajout de 5 cm³ d'une solution 3,2 N d'acide chlorhydrique dans l'éther éthylique, lavage à l'éther éthylique et séchage. On obtient 0,14 g de chlorhydrate de chloro-7 [(diméthylamino-2 phényl)-acétyl]-2 diphényl-4,4 perhydroisoindole- (3aR,7R,7aR) sous la forme de cristaux blancs fondant à 190°C.

### Exemple d'utilisation 6

A une solution de 0,43 g d'acide (diméthylamino-2 phényl) acétique dans 15 cm³ de dichlorométhane sec, refroidie à + 4°C, on ajoute 0,39 g de carbonyldiimidazole. On agite une heure à + 4°C, puis on ajoute une solution de 0,84 g de chlorhydrate de chloro-7 diphényl-4,4 perhydroisoindole-(3aR,7S,7aR) dans 10 cm³ de dichlorométhane sec, puis une solution de 0,34 cm³ de triéthylamine dans 10 cm³ de dichlorométhane sec. Le mélange réactionnel est agité à température ambiante pendant 20 heures, puis dilué par 100 cm³ de dichlorométhane, lavé par 50 cm³ d eau, puis par une solution aqueuse saturée en chlorure de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3 cm, hauteur 23 cm), en éluant sous une pression de 0,4 bar d'azote par un mélange d'acétate d'éthyle et de cyclohexane (25/75 en volumes) et en recueillant des fractions de 80 cm³. La fraction 2 est concentrée à sec sous pression réduite (2,7 kPa). Le produit obtenu sous forme de base, est converti en chlorhydrate par dissolution dans 4 cm³ d'acétonitrile, puis ajout de 6 cm³ d'une solution 3,2 N d'acide chlorhydrique dans l'éther éthylique, lavage à l'éther isopropylique et séchage. On obtient 0,08 g de chlorhydrate de chloro-7 [(diméthylamino-2 phényl)-acétyl]-2 diphényl-4,4 perhydroisoindole-(3aR,7S,7aR) sous la forme d'un solide beige.
Spectre infra-rouge (KBr, bandes caractéristiques, cm⁻¹): 3055, 3025, 2950, 1635, 1490, 1460, 1440, 760, 750, 700.
Spectre RMN du proton (DMSO-d₆) (à 403°K on observe le mélange des deux rotamères, DMSO-d₆ + CF₃COOD, signaux principaux): 3 et 3,13 (2s, 6H, N(CH₃)₂) : 4,54 et 4,63 (2mt, 1H, CHCl) : 7 à 7,8 (mt, 14H, aromatiques).

### Exemple d'utilisation 7

A une solution de 0,36 g d'acide (méthoxy-2 phényl)-2 propionique-(S) dans 20 cm³ de dichlorométhane sec, refroidie à +4°C, on ajoute 0,32 g de carbonyldiimidazole. On agite une heure à +4°C, puis on ajoute une solution de 0,67 g de chlorhydrate de diphényl-4,4 fluoro-7 perhydroisoindole-(3aR,7R,7aR) dans 20 cm³ de dichlorométhane sec et 0,28 cm³ de triéthylamine. Le mélange réactionnel est agité à température ambiante pendant 20 heures, dilué dans 200 cm³ de dichlorométhane puis lavé par 50 cm³ d'eau, séché sur sulfate de magnésium, filtré et concentré à sec sous pression réduite (2,7 kPa). Le résidu est chromatographié sur colonne de gel de silice (granulométrie 0,04-0,06 mm diamètre 3 cm, hauteur 20 cm), en éluant sous une pression de 0,4 bar d'azote par un mélange d'acétate d'éthyle et de cyclohexane (60/40 en volumes) et en recueillant des fractions de 20 cm³. Les fractions 10 à 15 sont réunies et concentrées à sec sous pression réduite (2,7 kPa). Le résidu est cristallisé dans 0,6 cm³ d'oxyde d'isopropyle. Les cristaux obtenus sont essorés, lavés par de l'oxyde d'isopropyle, puis séchés. On obtient 0,19 g de diphényl-4,4 fluoro-7 [(méthoxy-2 phényl)-2 propionyl-(S)]-2 perhydroisoindole-(3aR,7R,7aR) sous forme de cristaux blancs fondant à 195°C.

L'acide (méthoxy-2 phényl)-2 propionique-(S) peut être obtenu de la manière suivante :
L'acide (méthoxy-2 phényl) -2 propionique-(S) peut être préparé, par analogie avec les méthodes décrites par D.A Evans et coll., Tetrahedron, 44, 5525, (1988), selon le mode opératoire suivant: A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. [α]_{D}²⁰ = +84,6° (c=1; CHCl₃).

La méthyl-4 phényl-5 [(méthoxy-2 phényl)-2-(S)-propionyl]-3 oxazolidinone-2-(4S,5S) peut être obtenue de la manière suivante :
A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :
A une suspension de 1,89 d'hydrure de sodium (dispersion à 80% dans la vaseline) dans 200 cm³ de tétrahydrofuranne sec on ajoute à température ambiante 9,38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35,27 cm³ de butyllithium 1,6 M dans l'hexane à une solution refroidie à -78°C de 10 g de méthyl-4 phényl-5 oxazolidinone-2-(4S,5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle ; après décantation la phase organique est lavée deux fois par 100 cm³ d'eau, puis deux fois par 100 cm³ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 cm³. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

### Exemple d'utilisation 8

En opérant selon le mode opératoire de l'exemple d'utilisation 9 ci-après à partir de 0,77 g d'acide diméthylamino-2 phénylacétique et de 1,50 g de chlorhydrate de diphényl-4,4 difluoro-7,7 perhydroisoindole-(3aRS,7aRS) on obtient 1,29 g de [(diméthyl-amino-2 phényl)acétyl]-2 diphényl-4,4 difluoro-7,7 perhydro-isoindole-(3aRS,7aRS) sous la forme d'un solide blanc fondant à 189°C.

### Exemple d'utilisation 9

A une solution de 0,52 g d'acide diméthylamino-2 phénylacétique dans 20 cm³ de dichlorométhane sec, on ajoute 0,49 g de N,N'-carbonyldiimidazole. On agite 30 minutes à +5°C puis on ajoute une solution de 0,93 g de chlorhydrate de diphényl-7,7 perhydroisoindolol-4 -(3aR,4S,7aR) et de 0,84 cm³ de triéthylamine dans 10 cm³de dichlorométhane. Le mélange réactionnel est agité pendant 2 heures à +5°C puis lavé par 10 cm³d'eau, séché sur sulfate de magnésium, filtré et concentré & sec sous pression réduite (2,7 kPa). Le résidu obtenu est chromatographié sur une colonne de gel de silice (0,04 mm-0,06 mm, diamètre 2 cm, hauteur 35 cm), en éluant par de l'acétate d'éthyle et en recueillant des fractions de 30 cm³. Les fractions 8 à 27 sont réunies, concentrées à sec sous pression réduite (2,7 kPa). Le résidu est cristallisé dans un mélange de 4 cm³ d'acétonitrile et de 20 cm³ d'éther éthylique. Les cristaux sont essorés, séchés sous pression réduite (2,7 kPa). On obtient 0,70 g de [(diméthylamino-2 phényl)acétyl]-2 diphényl-7,7 perhydroisoindolol-4-(3aR,4S,7aR), sous la forme d'un solide blanc fondant à 160°C, [α]²⁰D = -162° (c = 0,5, méthanol).

### Exemple d'utilisation 10

En opérant selon le mode opératoire de l'exemple d'utilisation 9 à partir de 0,26 g d'acide diméthylamino-2 phénylacétique et de 0,50g de chlorhydrate de diphényl-7,7 perhydroisoindolol-4- (3aR,4R,7aR), on obtient 0,21 g de [(diméthylamino-2 phényl) acétyl]-2 diphényl-7,7 perhydroisoindolol-4-(3aR,4R,7aR), sous la forme d'un solide blanc fondant à 204°C, [α]²⁰D = -212° (c = 0,5, méthanol).

### Exemple d'utilisation 11

A une solution de 0,86 g de bromhydrate d'acide (pyrrolidino-2)-phényl-acétique dans 20 cm³ de dichlorométhane sec, refroidie à + 4°C, on ajoute 0,42 cm³ de triéthylamine et 0,49 g de carbonyldiimidazole. On agite une heure à + 4°C, puis on ajoute une solution de 1 g de chlorhydrate de diphényl-7,7 perhydro-isoindolol-4-(3aR,4S,7aR) et 0,42 cm³ de triéthylamine dans 10 cm³ de dichlorométhane sec. Le mélange réactionnel est agité à température ambiante pendant 24 heures, puis puis lavé deux fois par 10 cm³ d'eau, puis par une solution aqueuse d'hydrogénocarbonate de sodium, séché sur sulfate de magnésium, filtré et concentré à sec sous pression réduite (2,7 kPa). Le produit, obtenu sous forme de base, est converti en chlorhydrate par dissolution dans la quantité minimale d'acétone et traitement par une solution d'acide chlorhydrique dans l'éther éthylique et ajout d'éther éthylique. Le solide obtenu est trituré dans l'éther éthylique, puis séché. On obtient 0,2 g de chlorhydrate de diphényl-7,7 [(pyrrolidino-2 phényl) acétyl]-2 perhydroisoindolol-4-(3aR,4S,7aR) sous forme de solide beige.
Spectre infra-rouge (KBr, bandes caractéristiques, cm⁻¹): 3085, 3050, 3025, 2945, 2880, 2750, 2250, 1640, 1600, 1495, 1445, 1060, 755, 730, 700.
Spectre RMN du proton (DMSO-d₆): 0,92 et 1,72 (2 mt, 2x1H, CH₂- en 5) ; 2,17 (mt, 4H, 2 CH₂ en 3 et 4 du pyrrolidino) ; 7 à 7,8 (mt, 14H, aromatiques).

### Exemple d'utilisation 12

En opérant comme décrit précédemment dans l'exemple 9, à partir de 1,82 g d'acide (méthoxy-2 phényl)acétique et de 3,29 g de chlorhydrate de diphényl-7,7 perhydroisoindolol-4-(3aR,4S,7aR), on obtient 3,9 g de [(méthoxy-2 phényl)acétyl]-2 diphényl-7,7 perhydroisoindolol-4-(3aR,4S,7aR) sous la forme d'un solide blanc, fondant à 246°C. [α]²⁰D = -174° (c = 0,37; méthanol)

### Exemple d'utilisation 13

A une solution de 0,41 g d'acide (méthoxy-2 phényl)-2 propionique-(S) dans 15 cm³ de dichlorométhane sec, refroidie à + 4°C, on ajoute 0,37 g de carbonyldiimidazole. On agite une heure à + 4°C, puis on ajoute une solution de 0,75 g de chlorhydrate de diphényl-7,7 perhydroisoindolol-4-(3aR,4S,7aR). Le mélange réactionnel est agité à température ambiante pendant 20 heures, puis puis lavé deux fois par 10 cm³ d eau, séché sur sulfate de magnésium, filtré et concentré à sec sous pression réduite (2,7 kPa). Le résidu est chromatographié sur colonne de gel de silice (granulométrie 0,04-0,06 mm diamètre 3,6 cm, hauteur 37 cm), en éluant sous une pression de 0,5 bar d'azote par un mélange d'acétate d'éthyle et de cyclohexane ( 50/50 en volumes) et en recueillant des fractions de 50 cm³. Les fractions 21 à 41 sont réunies et concentrées à sec sous pression réduite (2,7 kPa). Le résidu est trituré dans de l'oxyde d'isopropyle puis séché. On obtient 0,3 g de diphényl-7,7 [(méthoxy-2 phényl)-2 propionyl-(S)]-2 perhydroisoindolol-4-(3aR,4S,7aR) sous la forme d'une meringue blanche.
Spectre infra-rouge (KBr, bandes caractéristiques, cm⁻¹): 3090, 3060, 3030, 2940, 2875, 2840, 1630, 1600, 1495, 1445, 1245, 1060, 755, 730, 700.
Spectre RMN du proton (DMSO-d₆) (à température ambiante, on observe le mélange des deux rotamères): 0,9-1,8 (mt, 2H, CH₂ en 5); 1,14 et 1,23 (2d, J=7, 3H, CH₃); 3,55 et 3,65 (2s, 3H, OCH₃); 3,85 et 4,23 (2mt, 1H, -COCHCH₃-); 6,8 à 7,5 (mt, 14H, aromatiques).

### Exemple d'utilisation 14

A une solution refroidie à + 10°C de 1 g de diphényl-7,7 perhydroisoindolol-4-(3aR,4S,7aR), 0,97 g d'acide (diméthylamino-3 propoxy)-2 phénylacétique, 0,05 g d'hydroxy-1 benzotriazole dans 50 cm³ de dichlorométhane on ajoute 0,766 g de (diméthylamino-3 propyl)-1 éthyl-3 carbodiimide. Le mélange réactionnel est agité 90 minutes 20°C puis lavé 2 fois Par 50 cm³ d eau et par 50 cm³ de solution saturée de chlorure de sodium. La phase organique est séchée sur sulfate de magnésium et concentrée à sec sous pression réduite (2,7 kPa). Le résidu est chromatographié sur une colonne de gel de silice (granulométrie 0,2-0,063 mm, diamètre 2,9 cm, hauteur 23 cm) en éluant sous une pression de 0,7 bar d'azote par des mélanges de dichloro-1,2 éthane et de méthanol (1 litre à 90/10 en volumes et 1,5 litre à 70/30 en volume) et en recueillant des fractions de 25 cm³. Les fractions 10 à 84 sont réunies et concentrées à sec sous pression réduite (2,7 kPa) pour donner 1,1 g de {[(diméthylamino-3 propoxy)-2 phényl]acétyl}-2 diphényl-7,7 perhydroisoindolol-4-(3aR,4S,7aR) sous la forme d'une meringue crème.
Spectre infra-rouge (KBr , bandes caractéristiques, cm⁻¹): 3080, 3050, 3020, 2940, 2870, 2815, 2765, 1635, 1600, 1490, 1455, 1445, 1245, 1065, 750, 730, 700.
Spectre RMN du proton (DMSO-d₆) à 433°K = 1,06 et 1,76 (2mt, 2x1H, CH₂ en 5) ; 2,27 (s, 6H, H(CH₃)₂) ; 3,9 (d, J=11, 1H, 1H du CH₂ en 3) ; 6,8 à 7,5 (mt, 14H, aromatiques)
Une solution de 100 g d'acide hydroxy-2 phénylacétique, 75 cm³ d alcool benzylique et 0,5 g d'acide paratoluène sulfonique dans 1400 cm³ de toluène est chauffée au reflux pendant 2 heures en éliminant l'eau formée. Après refroidissement, traitement par 3 g de noir animal et filtration, le mélange réactionnel est concentré à 150 cm³ et additionné de 300 cm³ d'oxyde d'isopropyle. Les cristaux obtenus par refroidissement à 0°C sont essorés lavés et séchés pour donner 82,5 g d'hydroxy-2 phénylacétate de benzyle. A une solution 153 g de cet ester dans un mélange de 500 cm³ de dibromo-1,3 propane et 2500 cm³ d'acétonitrile on ajoute 174 g de carbonate de potassium et l'on chauffe le mélange 17 heures au reflux. Le mélange réactionnel est refroidi, filtré et concentré à sec sous pression réduite (2,7 kPa). Le résidu est repris par 500 cm³ d'acétate d'éthyle et la phase organique est lavée par 2 fois par 400 cm³ d'eau et 2 fois par 250 cm³ de solution saturée de chlorure de sodium puis séchée et concentrée à sec sous pression réduite (2,7 kPa). Le résidu est chromatographié sur une colonne de gel de silice (granulométrie 0,2-0,063 mm, diamètre 9 cm, hauteur 55 cm) en éluant par un mélange de cyclohexane et d'acétate d'éthyle (95/5 en volumes) et en recueillant des fractions de 500 cm³. Les fractions 12 à 18 sont réunies et concentrées à sec sous pression réduite (2,7 kPa) pour donner 90 g de (bromo-3 propoxy)-2 phénylacétate de benzyle sous la forme d'une huile jaune. Une solution de 40 g de ce produit dans 500 cm³ d'acétonitrile est chauffée en autoclave avec 27 g d'iodure de sodium et 90 g de diméthylamine pendant 16 heures à 80°C - Le mélange réactionnel est refroidi, filtré et concentré à sec sous pression réduite (2,7 kPa). Le résidu est purifié par passage acide-base pour donner 29,3 g de (diméthylamino-3 propoxy)-2 phénylacétate de benzyle sous la forme d'une huile jaune. L'hydroyènation de cet ester à pression ordinaire à 40°C dans l'acétate d'éthyle en présence d'hydroxyde de palladium puis cristallisation dans l'acétate d'éthyle conduisent à 17,5 g d'acide (diméthylamino-3 propoxy)-2 phénylacétique sous la forme de cristaux blanc fondant à 98°C

### Exemple d'utilisation 15

A une solution de 0,41 g de tétrafluoroborate de (méthoxy-2 phényl)-acétimidate d'éthyle dans 10 cm³ de dichlorométhane sec, on ajoute une solution de 0,6 g de chlorhydrate de diphényl-4,4 fluoro-7 perhydroisoindole-(3aR,7R,7aR) et de 0,51 cm³ de triéthylamine dans 10 cm³ de dichlorométhane sec. Le mélange réactionnel est chauffé 3 heures au reflux. Il est ensuite traité, après retour à tempétature ambiante, par 5 cm³ d'une solution aqueuse de carbonate de potassium à 10%; la phase organique est lavée par 10 cm³ d'eau distillée, séchée sur sulfate de magnésium et concentrée à sec sous pression réduite (2,7 kPa) - Le résidu est chromatographié sur colonne de gel de silice (granulométrie 0,04-0,06 mm, diamètre 2 cm, hauteur 20 cm), en éluant sous une pression de 0,6 bar d'azote par un mélange d'acétate d'éthyle, d'acide acétique et d'eau (15/1/1 en volumes) et en recueillant des fractions de 25 cm³. Les fractions 24 à 38 sont réunies et concentrées à sec sous pression réduite (2,7 kPa). Le résidu est repris par 40 cm³ de dichlorométhane, lavé par 10 cm³ de solution aqueuse saturée de carbonate de potassium, puis par 10 cm³ de solution aqueuse saturée de chlorure de sodium. La phase organique est séchée sur sulfate de magnésium et concentrée à sec sous pression réduite (2,7 kPa). Le résidu est cristallisé dans l'oxyde d'isopropyle. Les cristaux sont essorés et séchés. On obtient 0,18 g de diphényl-4,4 fluoro-7 [imino-1 (méthoxy-2 phényl] -2 éthyl]-2 perhydro-isoindole-(3aR,7R,7aR) sous la forme de cristaux blancs fondant à 184°C avec décomposition.

### Exemple d'utilisation 16

A une solution de 2 g de diphényl-7,7 perhydro-isoindolol-4-(3aR,4S,7aR) dans 30 cm³ de dichlorométhane sec, on ajoute une solution de 1,56 g de tétrafluoroborate de (méthoxy-2 phényl)-acétimidate d'éthyle et de 0,96 cm³ de triéthylamine dans 20 cm³ de dichlorométhane sec, puis on porte le mélange réactionnel au reflux pendant 2 heures. On ajoute ensuite 10 cm³ d une solution aqueuse à 10% de carbonate de potassium, on décante, puis on lave la phase organique Par 20 cm³ d'eau, on sèche sur sulfate de magnésium, on filtre et concentre à sec sous pression réduite (2,7 kPa). Le résidu est chromatographié sur colonne d'alumine (diamètre 3,6 cm, hauteur 31 cm) , en éluant sous une pression de 0,1 bar d' azote par un mélange de dichlorométhane et de méthanol (95/5 en volumes) et en recueillant des fractions de 50 cm³. Les fractions 5 à 30 sont réunies et concentrées à sec sous pression réduite (2,7 kPa). Le solide obtenu est lavé à l'oxyde d'isopropyle, essoré et séché. On obtient 1,4 g de diphényl-7,7 [imino-1 (méthoxy-2 phényl)-2 éthyl]-2 perhydroisoindolol-4-(3aR,4S,7aR) sous la forme de cristaux blancs, fondant à 105°C avec décomposition.

## Revendications

1. Un dérivé de perhydroisoindole caractérisé en ce qu'il répond à la formule générale : 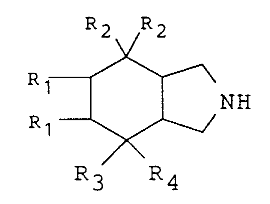 dans laquelle
- les radicaux R₁ sont identiques et représentent des atomes d'hydrogène ou forment ensemble une liaison,
- les symboles R₂ sont identiques et représentent des radicaux phényle éventuellement substitués par un atome d'halogène ou par un radical méthyle en position 2 ou 3,
- le symbole R₃ représente un atome d'halogène ou un radical hydroxy et
- le symbole R₄ représente un atome d'hydrogène ou simultanément à R₃ représente un atome d'halogène,
sous ses formes stéreoisomères ou leurs mélanges, ainsi que ses sels.

2. Un dérivé de perhydroisoindole selon la revendication 1, caractérisé en ce que :
- les radicaux R₁ sont des atomes d'hydrogène,
- les symboles R₂ sont identiques et représentent des radicaux phényle,
- le symbole R₃ représente un atome de fluor ou de chlore ou un radical hydroxy et
- le symbole R₄ représente un atome d'hydrogène ou simultanément à R₃ représente un atome de fluor,
sous ses formes stéreoisomères ou leurs mélanges, ainsi que ses sels.

3. Un dérivé de perhydroisoindole selon la revendication 1 caractérisé en ce qu'il s'agit du diphényl-7,7 perhydroisoindolol-4, sous ses formes stéreoisomères ou leurs mélanges, ainsi que ses sels.

4. Un dérivé de perhydroisoindole selon la revendication 1 caractérisé en ce qu'il s'agit du diphényl-4,4 fluoro-7 perhydroisoindole, sous ses formes stéreoisomères ou leurs mélanges, ainsi que ses sels.

5. Un dérivé de perhydroisoindole selon la revendication 1 caractérisé en ce qu'il s'agit du diphényl-4,4 difluoro-7,7 perhydroisoindole, sous ses formes stéreoisomères ou leurs mélanges, ainsi que ses sels.

6. Un dérivé de perhydroisoindole selon la revendication 1 caractérisé en ce qu'il s'agit du chloro-7 diphényl-4,4 perhydroisoindole, sous ses formes stéreoisomères ou leurs mélanges, ainsi que ses sels^{.}

7. Procédé de préparation d'un dérivé de per-hydroisoindole selon la revendication 1 pour lequel R₃ est un atome d'halogène et R₄ est un atome d'hydrogène ou d'halogène, caractérisé en ce que l'on traite par un agent d'halogénation un dérivé de l'isoindole de formule générale : 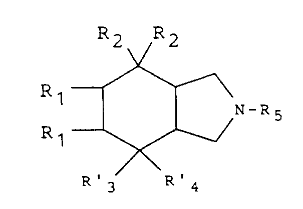 dans laquelle R₁ et R₂ sont définis comme précédemment dans la revendication 1, R₅ est un radical protecteur et, R'₃ est un radical hydroxy et R'₄ est un atome d'hydrogène si l'on veut obtenir un dérivé monohalogéné, ou R'₃ et R'₄ forment ensemble un radical oxo si l'on veut obtenir un dérivé dihalogéné, puis élimine le radical protecteur R₅ et transforme éventuellement le produit obtenu en un sel.

8. Procédé de préparation d'un dérivé de perhydroisoindole selon la revendication 1 pour lequel R₃ est un atome d'halogène et R₄ est un atome d'hydrogène, caractérisé en ce que l'on traite par un agent d'halogénation un dérivé de l'isoindole de formule générale : 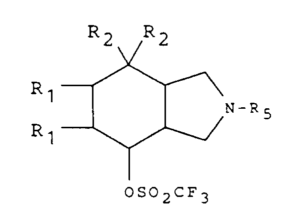 dans laquelle R₁ , R₂ et R₅ sont définis comme dans la revendication 2, puis élimine le radical protecteur R₅ et transforme éventuel-lement le produit obtenu en un sel.

9. Procédé de préparation d'un dérivé de perhydroisoindole selon la revendication 1 pour lequel R₃ est un radical hydroxy et R₄ est un atome d'hydrogène, caractérisé en ce que l'on réduit un dérivé de perhydroisoindolone de formule générale : 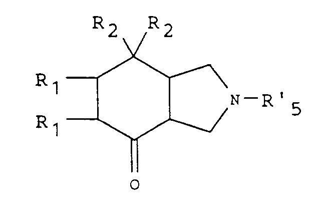 dans laquelle R₁ et R₂ sont définis comme dans la revendication 1 et R'₅ est défini comme R₅ ou représente un atome d'hydrogène, puis sépare éventuellement les isomères axial et équatorial et transforme éventuellement le produit obtenu en un sel.

10. Procédé de préparation d'un dérivé de perhydroisoindole selon la revendication 1 pour lequel R₃ est un radical hydroxy et R₄ est un atome d'hydrogène, caractérisé en ce que l'on élimine le radical protecteur du dérivé de perhydroisoindole de formule générale : 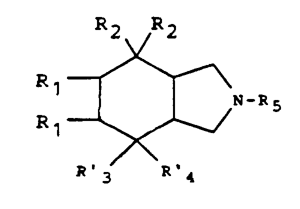 dans laquelle R₁ et R₂ sont définis comme précédemment dans la revendication 1, R₅ est un radical protecteur, R'₃ est un radical hydroxy et R'₄ est un atome d'hydrogène, pas transforme éventuellement le produit obtenu en un sel.

11. Utilisation d'un dérivé de l'isoindole selon la revendication 1 pour la préparation d'un produit de formule générale : 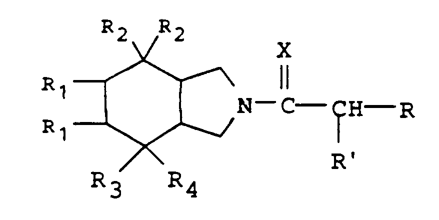 dans laquelle
- les radicaux R₁ sont identiques et représentent des atomes d'hydrogène ou forment ensemble une liaison,
- les symboles R₂ sont identiques et représentent des radicaux phényle éventuellement substitués par un atome d'halogène ou par un radical méthyle en position 2 ou 3,
- le symbole X représente un atome d'oxygène, ou un radical NH,
- le symbole R représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou, dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole R' représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,
- le symbole R₃ représente un atome d'halogène ou un radical hydroxy et
- le symbole R₄ représente un atome d'hydrogène ou simultanément à R₃, représente un atome d'halogène,
les radicaux alcoyle et acyle cités ci-dessus étant droits ou ramifiés et contenant 1 à 4 atomes de carbone, sous leurs formes stéréoisomères ou leurs mélanges, ainsi que leurs sels lorsqu'ils existent.

## Claims

1. A perhydroisoindole derivative characterized in that it corresponds to the general formula: 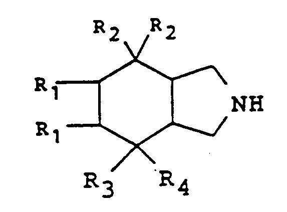 in which
- the radicals R₁ are identical and represent hydrogen atoms or together form a bond,
- the symbols R₂ are identical and represent phenyl radicals which are optionally substituted by a halogen atom or by a methyl radical in position 2 or 3,
- the symbol R₃ represents a halogen atom or a hydroxyl radical and
- the symbol R₄ represents a hydrogen atom or, simultaneously with R₃, represents a halogen atom,
in its stereoisomeric forms or mixtures thereof, as well as its salts.

2. A perhydroisoindole derivative according to Claim 1, characterized in that:
- the radicals R₁ are hydrogen atoms,
- the symbols R₂ are identical and represent phenyl radicals,
- the symbol R₃ represents a fluorine or chlorine atom or a hydroxyl radical, and
- the symbol R₄ represents a hydrogen atom or, simultaneously with R₃, represents a fluorine atom,
in its stereoisomeric forms or mixtures thereof, as well as its salts.

3. A perhydroisoindole derivative according to Claim 1, characterized in that it is 7,7-diphenyl-4-perhydroisoindolol, in its stereoisomeric forms or mixtures thereof, as well as its salts.

4. A perhydroisoindole derivative according to Claim 1, characterized in that it is 4,4-diphenyl-7-fluoroperhydroisoindole, in its stereoisomeric forms or mixtures thereof, as well as its salts.

5. A perhydroisoindole derivative according to Claim 1, characterized in that it is 4,4-diphenyl-7,7-difluoroperhydroisoindole, in its stereoisomeric forms or mixtures thereof, as well as its salts.

6. A perhydroisoindole derivative according to Claim 1, characterized in that it is 7-chloro-4,4-diphenylperhydroisoindole, in its stereoisomeric forms or mixtures thereof, as well as its salts.

7. Method of preparing a perhydroisoindole derivative according to Claim 1, for which R₃ is a halogen atom and R₄ is a hydrogen or halogen atom, characterized in that an isoindole derivative of general formula: 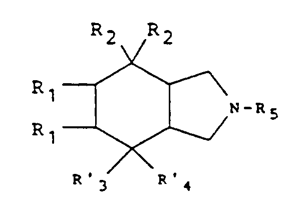 in which R₁ and R₂ are as defined in Claim 1 above, R₅ is a protective radical and, R'₃ is a hydroxyl radical and R'₄ is a hydrogen atom if it is desired to obtain a monohalogenated derivative, or R'₃ and R'₄ together form an oxo radical if it is desired to obtain a dihalogenated derivative, is treated with a halogenating agent and then the protective radical R₅ is removed and the product obtained is optionally converted to a salt.

8. Method of preparing a perhydroisoindole derivative according to Claim 1, for which R₃ is a halogen atom and R₄ is a hydrogen atom, characterized in that an isoindole derivative of general formula: 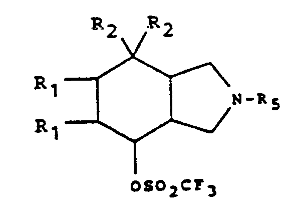 in which R₁, R₂ and R₅ are as defined in Claim 2, is treated with a halogenating agent and then the protective radical R₅ is removed and the product obtained is optionally converted to a salt.

9. Method of preparing a perhydroisoindole derivative according to Claim 1, for which R₃ is a hydroxyl radical and R₄ is a hydrogen atom, characterized in that a perhydroisoindolone derivative of general formula: 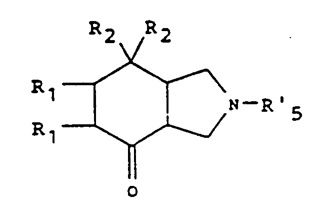 in which R₁ and R₂ are as defined in Claim 1 and R'₅ is defined as R₅ or represents a hydrogen atom, is reduced and then the axial and equatorial isomers are optionally separated and the product obtained is optionally converted to a salt.

10. Method of preparing a perhydroisoindole derivative according to Claim 1, for which R₃ is a hydroxyl radical and R₄ is a hydrogen atom, characterized in that the radical protecting the perhydroisoindole derivative of general formula: 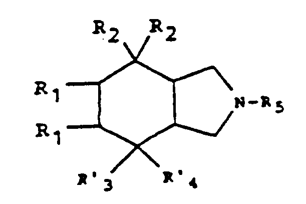 in which R₁ and R₂ are as defined in Claim 1 above, R₅ is a protective radical, R'₃ is a hydroxyl radical and R'₄ is a hydrogen atom, is removed and then the product obtained is optionally converted to a salt.

11. Use of an isoindole derivative according to Claim 1, for the preparation of a product of general formula: 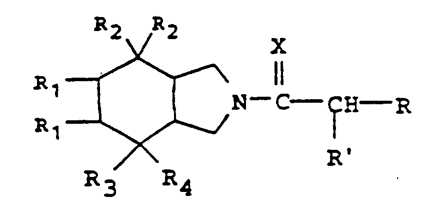 in which
- the radicals R₁ are identical and represent hydrogen atoms or together form a bond,
- the symbols R₂ are identical and represent phenyl radicals which are optionally substituted by a halogen atom or by a methyl radical in position 2 or 3,
- the symbol X represents an oxygen atom or an NH radical,
- the symbol R represents a phenyl radical which is optionally substituted by one or more halogen atoms or hydroxyl or alkyl radicals which may be optionally substituted (by halogen atoms or amino, alkylamino or dialkylamino radicals) alkoxy or alkylthio radicals which may be optionally substituted [by hydroxyl, amino, alkylamino or dialkylamino radicals optionally substituted (by phenyl, hydroxyl or amino radicals) or by dialkylamino radicals whose alkyl parts form with the nitrogen atom to which they are attached, a heterocycle with 5 to 6 members which may contain another heteroatom chosen from oxygen, sulphur or nitrogen, optionally substituted by an alkyl, hydroxyl or hydroxyalkyl radical)], or which is substituted by amino, alkylamino or dialkylamino radicals whose alkyl parts may form with the nitrogen atom to which they are attached, a heterocycle as defined above, or represents a cyclohexadienyl, naphthyl or a saturated or unsaturated, mono- or polycyclic heterocyclic radical containing 5 to 9 carbon atoms and one or more heteroatoms chosen from oxygen, nitrogen or sulphur,
- the symbol R' represents a hydrogen or halogen atom or a hydroxyl, alkyl, aminoalkyl, alkylaminoalkyl, dialkylaminoalkyl, alkoxy, alkylthio, acyloxy, carboxyl, alkoxycarbonyl, dialkylaminoalkoxycarbonyl, benzyloxycarbonyl, amino, acylamino or alkoxycarbonylamino radical,
- the symbol R₃ represents a halogen atom or a hydroxyl radical and
- the symbol R₄ represents a hydrogen atom or simultaneously with R₃, represents a halogen atom,
the abovementioned alkyl and acyl radicals being linear or branched and containing 1 to 4 carbon atoms in their stereoisomeric forms or mixtures thereof, as well as their salts when these exist.

## Patentansprüche

1. Perhydroisoindolderivat, dadurch gekennzeichnet, daß es der allgemeinen Formel 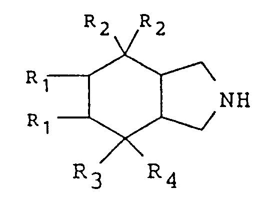 entspricht, worin
- die Reste R₁ identisch sind und Wasserstoffatome bedeuten oder gemeinsam eine Bindung bilden,
- die Symbole R₂ identisch sind und gegebenenfalls durch ein Halogenatom oder durch einen Methylrest in 2- oder 3-Stellung substituierte Phenylreste darstellen,
- das Symbol R₃ ein Halogenatom oder einen Hydroxyrest wiedergibt und
- das Symbol R₄ für ein Wasserstoffatom steht oder gleichzeitig mit R₃ ein Halogenatom bedeutet,
in ihren stereoisomeren Formen oder in Form von deren Gemischen sowie deren Salze.

2. Perhydroisoindolderivat gemäß Anspruch 1, dadurch gekennzeichnet, daß
- **die Reste R₁ Wasserstoffatome sind,**
- die Symbole R₂ identisch sind und Phenylreste bedeuten,
- das Symbol R₃ für ein Fluor- oder Chloratom oder einen Hydroxyrest steht und
- das Symbol R₄ ein Wasserstoffatom bedeutet oder gleichzeitig mit R₃ für ein Fluoratom steht,
in ihren stereoisomeren Formen oder in Form von deren Gemischen sowie deren Salze.

3. Perhydroisoindolderivat gemäß Anspruch 1, dadurch gekennzeichnet, daß es sich um 7,7-Diphenyl-4-perhydroisoindolol in seinen stereoisomeren Formen oder deren Gemische sowie seine Salze handelt.

4. Perhydroisoindolderivat gemäß Anspruch 1, dadurch gekennzeichnet, daß es sich um 4,4-Diphenyl-7-fluorperhydroisoindol in seinen stereoisomeren Formen oder deren Gemische sowie seine Salze handelt.

5. Perhydroisoindolderivat gemäß Anspruch 1, dadurch gekennzeichnet, daß es sich um 4,4-Diphenyl-7,7-difluorperhydroisoindol in seinen stereoisomeren Formen oder deren Gemische sowie seine Salze handelt.

6. Perhydroisoindolderivat gemäß Anspruch 1, dadurch gekennzeichnet, daß es sich um 7-Chlor-4,4-diphenylperhydroisoindol in seinen stereoisomeren Formen oder deren Gemische sowie seine Salze handelt.

7. Verfahren zur Herstellung eines Perhydroisoindolderivats gemäß Anspruch 1, worin R₃ ein Halogenatom bedeutet und R₄ für ein Wasserstoff- oder Halogenatom steht, dadurch gekennzeichnet, daß man mit einem Halogenierungsmittel ein Isoindolderivat der allgemeinen Formel 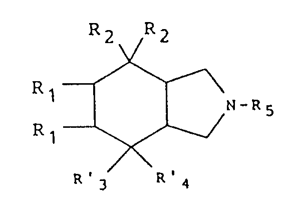 behandelt,
worin R₁ und R₂ wie vorstehend in Anspruch 1 definiert sind, R₅ für eine Schutzgruppe steht und R'₃ eine Hydroxygruppe wiedergibt und R'₄ ein Wasserstoffatom bedeutet, wenn man ein monohalogeniertes Derivat erhalten möchte, oder R'₃ und R'₄ gemeinsam einen Oxorest bedeuten, wenn man ein dihalogeniertes Derivat erhalten möchte, hiernach die Schutzgruppe R₅ entfernt und gegebenenfalls das erhaltene Produkt in ein Salz überführt.

8. Verfahren zur Herstellung eines Perhydroisoindolderivats gemäß Anspruch 1, worin R₃ ein Halogenatom bedeutet und R₄ für ein Wasserstoffatom steht, dadurch gekennzeichnet, daß man mit einem Halogenierungsmittel ein Isoindolderivat der allgemeinen Formel 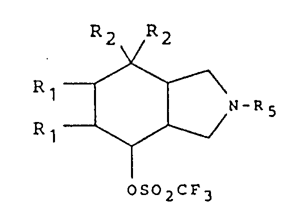 behandelt, worin R₁, R₂ und R₅ wie in Anspruch 2 definiert sind, hiernach die Schutzgruppe R₅ entfernt und gegebenenfalls das erhaltene Produkt in ein Salz überführt.

9. Verfahren zur Herstellung eines Perhydroisoindolderivats gemäß Anspruch 1, worin R₃ für eine Hydroxygruppe steht und R₄ ein Wasserstoffatom bedeutet, dadurch gekennzeichnet, daß man ein Perhydroisoindolonderivat der allgemeinen Formel 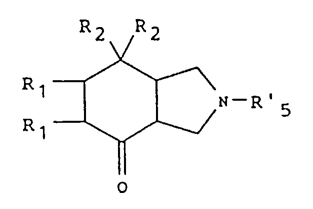 reduziert, worin R₁ und R₂ wie in Anspruch 1 definiert sind und R'₅ wie R₅ definiert ist oder ein Wasserstoffatom bedeutet, hiernach gegebenenfalls die axialen und äquatorialen Isomeren trennt und gegebenenfalls das erhaltene Produkt in ein Salz überführt.

10. Verfahren zur Herstelllung eines Perhydroisoindolderivats gemäß Anspruch 1, worin R₃ eine Hydroxygruppe bedeutet und R₄ für ein Wasserstoffatom steht, dadurch gekennzeichnet, daß man die Schutzgruppe des Perhydroisoindolderivats der allgemeinen Formel 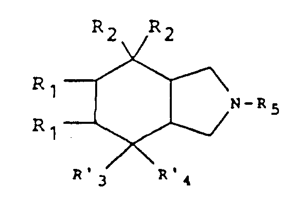 entfernt, worin R₁ und R₂ wie vorstehend in Anspruch 1 definiert sind, R₅ eine Schutzgruppe bedeutet, R'₃ eine Hydroxygruppe bedeutet und R'₄ ein Wasserstoffatom ist, und hiernach gegebenenfalls das erhaltene Produkt in ein Salz überführt.

11. Verwendung eines Isoindolderivats gemäß Anspruch 1 zur Herstellung eines Produkts der allgemeinen Formel 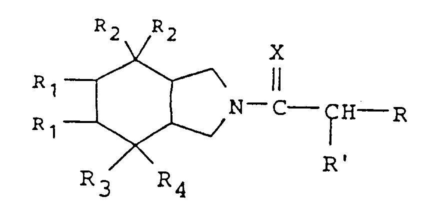 worin
- die Reste R₁ identisch sind und Wasserstoffatome darstellen oder gemeinsam eine Bindung bilden,
- die Symbole R₂ identisch sind und gegebenenfalls durch ein Halogenatom oder durch einen Methylrest in 2- oder 3-Stellung substituierte Phenylreste bedeuten,
- das Symbol X ein Sauerstoffatom oder einen Rest NH wiedergibt,
- das Symbol R einen gegebenenfalls durch ein oder mehrere Halogenatome oder Hydroxyreste substituierten Phenylrest, Alkyl, das gegebenenfalls substituiert sein kann (durch Halogenatome oder Amino-, Alkylamino- oder Dialkylaminoreste), Alkoxy oder Alkylthio, die gegebenenfalls substituiert sein können [durch Hydroxy-, Amino-, Alkylamino- oder Dialkylaminoreste, gegebenfalls substituiert (durch Phenyl-, Hydroxy- oder Aminoreste), oder Dialkylamino, dessen Alkylteile mit dem Stickstoffatom, an das sie gebunden sind, einen fünf- oder sechsgliedrigen Heterocyclus bilden, der ein Heteroatom, ausgewählt unter Sauerstoff, Schwefel oder Stickstoff, enthalten kann, und gegebenenfalls durch einen Alkyl-, Hydroxy-, Hydroxyalkylrest substituiert ist)], oder substituiert durch Amino-, Alkylamino-, Dialkylaminoreste, deren Alkylteile mit dem Stickstoffatom, an das sie gebunden sind, einen Heterocyclus, wie vorstehend definiert, bilden können, bedeutet, oder einen Cyclohexadienyl-, Naphthyl- oder mono- oder polycyclischen gesättigten oder ungesättigten Heterocyclus mit 5 bis 9 Kohlenstoffatomen und einem oder mehreren Heteroatomen, ausgewählt unter Sauerstoff, Stickstoff oder Schwefel, bedeutet, das Symbol R' ein Wasserstoff- oder Halogenatom oder einen Hydroxy-, Alkyl-, Aminoalkyl-, Alkylaminoalkyl-, Dialkylaminoalkyl-, Alkoxy-, Alkylthio-, Acyloxy-, Carboxy-, Alkoxycarbonyl-, Dialkylaminoalkoxycarbonyl-, Benzyloxycarbonyl-, Amino-, Acylamino- oder Alkoxycarbonylaminorest bedeutet,
- das Symbol R₃ für ein Halogenatom oder eine Hydroxygruppe steht und
- das Symbol R₄ ein Wasserstoffatom oder gleichzeitig mit R₃ ein Halogenatom bedeutet,
wobei die vorstehend genannten Alkyl- und Acylreste geradkettig oder verzweigt sind und 1 bis 4 Kohlenstoffatome aufweisen, in ihren stereoisomeren Formen oder deren Gemischen sowie deren Salzen, soweit sie existieren.
